# EUROPÄISCHE PATENTSCHRIFT

(11) **EP 1 485 345 B1**
(45) Veröffentlichungstag und Bekanntmachung des Hinweises auf die Patenterteilung: **25.06.2008**
(21) Anmeldenummer: 03714803.8
(22) Anmeldetag: 11.03.2003
(51) Int. Cl.: C07K 5/062, C07K 5/037, A61K 38/05, A61K 38/06

(54) **HEMMSTOFFE DER UROKINASE, IHRE HERSTELLUNG UND VERWENDUNG**
UROKINASE INHIBITORS, PRODUCTION AND USE THEREOF
INHIBITEURS DE L'UROKINASE, LEUR PRODUCTION ET LEUR UTILISATION

(30) Priorität: 11.03.2002 DE 10210592; 26.09.2002 DE 10245059; 28.12.2002 DE 10261435
(43) Veröffentlichungstag der Anmeldung: 15.12.2004
(73) Patentinhaber: Curacyte AG, 81675 München (DE)
(72) Erfinder: STÜRZEBECHER, Jörg, 99094 Erfurt (DE); STEINMETZER, Torsten, 07743 Jena (DE); SCHWEINITZ, Andrea, 07745 Jena (DE)
(74) Vertreter: Bösl, Raphael Konrad
(86) Internationale Anmeldenummer: PCT/EP2003/002489
(87) Internationale Veröffentlichungsnummer: WO 2003/076391

(56) Entgegenhaltungen:
- WO-A-02/14349
- DE-A- 10 029 014
- KÜNZEL S ET AL: "4-Amidinobenzylamine-Based Inhibitors of Urokinase" BIOORGANIC & MEDICINAL CHEMISTRY LETTERS, OXFORD, GB, Bd. 12, Nr. 4, 25. Februar 2002 (2002-02-25), Seiten 645-648, XP002193207 ISSN: 0960-894X in der Anmeldung erwähnt
- ZESLAWSKA E ET AL.: "Crystals of Urokinase Type Plasminogen Activator Complexes Reveal the Binding Mode of Peptidomimetic Inhibitors" JOURNAL OF MOLECULAR BIOLOGY, Bd. 328, Nr. 1, 18. April 2003 (2003-04-18), Seiten 109-118, XP002253026

## Beschreibung

Die Erfindung betrifft Hemmstoffe der Urokinase, ihre Herstellung und Verwendung zur Therapie, Prophylaxe und Diagnose eines Tumors, insbesondere zur Reduzierung der Bildung von Tumormetastasen.

Die Ausbreitung und Metastasierung solider Tumoren in umgebendem Gewebe wird durch ihre Fähigkeit, die extrazelluläre Matrix in der Umgebung der Tumorzelle abzubauen bzw. die Basalmembran zu durchdringen, ermöglicht. In diesem Prozess kommt neben verschiedenen Matrixmetalloproteinasen und Cathepsinen vor allem dem Plasminogenaktivator Urokinase (uPA) eine zentrale Bedeutung zu (P. Mignatti und D.B. Rifkin, Physiol. Rev. 73, 161-195, 1993). So bewirkt uPA die Aktivierung von Plasminogen; das entstehende Plasmin kann die Komponenten der extrazellulären Matrix (Fibrin, Fibronektin, Laminin, Proteoglykane u.a.) abbauen sowie Metalloproteasen und pro-Urokinase zu uPA aktivieren (U. Reuning et al., Int. J. Oncol. 13, 893-906, 1998).

Sowohl pro-Urokinase als auch uPA binden an den uPA-Rezeptor (uPAR), einen an der Zelloberfläche lokalisierten, spezifischen Rezeptor. Dadurch wird eine Verstärkung und Fokussierung der Aktivität von uPA und damit der Plasminogenaktivierung in der direkten Umgebung der Tumorzelle erreicht. Sowohl in zellbiologischen Studien als auch in Tiermodellen konnte die Bedeutung dieses zellassoxiierten Plasminogenaktivator-Systems für Tumorwachstum und -ausbreitung gezeigt werden. So wird das invasive Potential von Tumorzellen bei Hemmung der enzymatischen Aktivität von uPA durch die natürlichen Inhibitoren PAI-1 und PAI-2 herabgesetzt (J.-F. Cajot et al., Proc. Natl. Acad. Sci. USA 87, 6939-6943, 1990; M. Baker et al., Cancer Res. 50, 4876-4684, 1990). In Hühnerembryonen konnte durch Zugabe von Antikörpern gegen uPA die durch menschliche Karzinomzellen verursachte Bildung von Lungenmetastasen fast vollständig verhindert werden (L. Ossowski et al., Cell 35, 611-619,1983).

Die Faktoren des Plasminogenaktivator-Systems (uPA, uPAR, PA1-1 und PAI-2) sind in den letzten Jahren hinsichtlich ihrer klinischen Relevanz für die Prognose von Patienten mit soliden malignen Tumoren intensiv untersucht worden. Insbesondere der Gehalt von uPA im Gewebe verschiedener Tumoren erwies Sich als ein Prognosefaktor. So haben Patienten mit hohem uPA-Spiegel eine schlechtere Prognose als solche mit niedriger uPA-Konzentration im Tumor (M. Schmitt et al., Thromb. Haemost. 78, 285-296, 1997; R.W. Stephens et al., Breast Cancer Res. Treat. 52, 99-111, 1998). Auch eine erhöhte Konzentration an uPAR im Tumorgewebe korreliert mit einer schlechten Prognose (H. Pedersen et al., Cancer Res. 54, 4671-4675, 1994; C. Duggan et al., Int. J. Cancer 61, 597-600, 1995).

Aus den Befunden zum prognostischen Wert des uPA- und uPAR-Gehaltes im Tumorgewebe kann angenommen werden, dass synthetische uPA-Inhibitoren in der Lage sind, Invasion und Ausbreitung von Tumorzellen zu unterdrücken. Die Zahl der bisher bekannten uPA-Hemmstoffe ist allerdings relativ klein. Die Mehrzahl besitzt nur eine geringe Spezifität und Wirkungsstärke, wie es für verschiedene Benzamidin- und β-Naphthamidin-Derivate zutrifft (J. Stürzebecher und F. Markwardt, Pharmazie 33, 599-602, 1978). Das von Vassalli und Belin (FEBS Letters 214, 187-191, 1997) als uPA-Hemmstoff beschriebene Amilorid ist zwar ein spezifischer, aber schwacher Inhibitor von uPA (Kᵢ = 7 µM).

Stärker wirksame uPA-Inhibitoren wurden mit 4-substituierten Benzothiophen-2-carboxamidinen (Kᵢ = 0,16 µM für Verbindung B-623) gefunden. Hemmstoffe dieses Typs inaktivieren auch uPA, die an uPAR gebunden ist (M.J. Towle et al., Cancer Res. 53, 2553-2559, 1993). Die Benzothiophen-Derivate sind sehr spezifisch, ihre Hemmwirkung gegenüber Plasmin und dem Plasminogenaktivator vom Gewebetyp (tPA) ist gering, allerdings ist die Synthese von Verbindungen dieses Typs sehr aufwändig.

Eine vergleichbare Spezifität haben auch 4-Aminomethylphenylguanidin-Derivative, deren Hemmwirkung gegenüber uPA (Kᵢ = 2,4 µM für die wirksamste Verbindung) allerdings vergleichsweise gering ist (S. Sperl et al., Proc. Natl. Acad. Sci. USA 97, 5113-5118, 2000).

Im Gegensatz dazu erreichen Nα-Triisopropylphenylsulfonyl-3-amidinophenyl-alanin-Derivate mikromolare Kᵢ-Werte (0.41 µM für die wirksamste Verbindung), sind allerdings sehr unspezifische uPA-Hemmstoffe, mit gleicher oder stärkerer Wirkung werden Trypsin, Thrombin und Plasmin inhibiert (J. Stürzebecher et al., Bioorg. Med. Letters 9, 3147-3152, 1999). Sehr wirksame uPA-Hemmstoffe sind in der WO 99/05096 und WO 01/81314 mit weiterentwickelten β-Naphthamidinen offenbart. Es werden IC₅₀-Werte im nanomolaren Bereich beschrieben, allerdings keine Angaben zur Selektivität und der biologischen Wirksamkeit gemacht.

Bisher wurden nur wenige Peptide als uPA-Hemmstoffe beschrieben, die sich von der Substrat-Sequenz ableiten. Kettner und Shaw (Methods in Enzymology, 80, 826-842, 1981) beschrieben Chlormethylketone, die zwar uPA irreversibel hemmen, aber nicht für in vivo-Anwendung geeignet sind.

In der EP 18 32 71 sind Lysin-Derivate offenbart, die eine gewisse uPA Hemmung bewirken, allerdings auch andere vergleichbare Enzyme hemmen und damit nur sehr speziell bzw. eingeschränkt für medizinische Zwecke verwendbar sind. Gleiches gilt für die in der WO 95/17 8 85 als uPA-Inhibitoren beschriebenen niedermolekularen Polypeptide (ca. 50 Aminosäuren), die sich von natürlichen Hemmstoffen ableiten. Auf Grund ihres Peptidcharakters und der Molekülgröße ist ihr in vivo-Einsatz stark eingeschränkt. In jüngster Zeit wurden in WO 00/05245 Peptidylaldehyde mitgeteilt, die C-terminal ein Arginal und in P3 ein D-Serin enthielten und uPA sehr wirksam hemmten. Nach Acylierung des D-Ser-Hydroxyl konnte für die Leitverbindung iBuOCO-D-Ser-Ala-Arg-H nach s.c.-Gabe eine relative Bioverfügbarkeit von 87 % beobachtet werden (S. Y. Tamura et aL Bioorg. Med. Chem. Lett. 10, 983-987, 2000). In PCT/EP WO 01/96286 werden Hemmstoffe offenbart, die sich von acyliertem Amidinobenzylamin ableiten und neben einer natürlichen Aminosäure in P2 ein D-Serin oder eine vergleichbare unnatürliche Aminosäure in P3 enthalten. Verbindungen diese Typs hemmen Urokinase (Kᵢ = 36 nM für die wirksamste Verbindung) sehr effektiv. Allerdings haben Verbindungen diese Typs nur unzureichende pharmakokinetische Eigenschaften für eine Anwendung in vivo; sie werden nach oraler Gabe kaum resorbiert und im Versuchstier nach i.v.-Gabe sehr schnell aus der Zirkulation eliminiert (Künzel et al., Bioorg. Med. Chem. Lett. 12, 645-648 (2002)). In WO 02/14349 werden weitere nicht-kovalent bindende Urokinase-Hemmstoffe beschrieben, die neben den bereits in WO 01/96286 beschriebenen acylierten Amidinobenzylaminen z.B. acyliertes Guanidinobenzylamin, 2-Amidino-5-Aminomethylthiophen und andere Arginin-Mimetika als P1-Rest besitzen.

Der Erfindung liegt daher die Aufgabe zu Grunde, einen auch für therapeutische Anwendungen geeigneten Wirkstoff anzugeben, der Urokinase mit hoher Aktivität hemmt und der nach i.v.- oder s.c.-Gabe möglichst lange im Körper zirkuliert.

Überraschend wurde gefunden, dass acyliertes Amidinobenzylamin gemäß der im Patentanspruch 1 angeführten allgemeinen Formel I 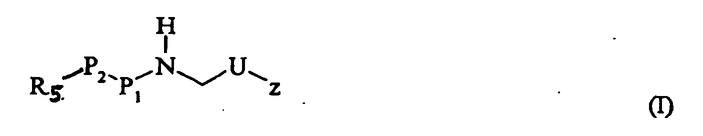 wobei 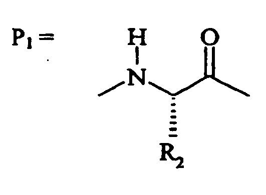 und 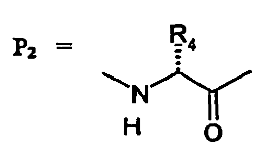 ist,
insbesondere Verbindungen des 4-Amidinobenzylamins, bei denen R₂ und R₄ natürliche und/oder unnatürliche Aminosäuren ergeben, dann sowohl Urokinase sehr wirksam inaktivieren als auch insbesondere nach i.v.- oder s.c.-Gabe langsam aus der Zirkulation eliminiert werden, wenn neben der Amidinofunktion weitere geladene Gruppen, vorzugsweise Carboxyl, Amino, Amidino, Hydroxyamidino, Amidrazono oder Guanidino eingeführt werden. Die. Carboxylgruppen können auch geschützt in Form ihrer Ester vorliegen, wobei bevorzugt Ethylester Verwendung finden. Diese Ester werden in vivo teilweise in die freien Säuren umgewandelt.

Das oben Gesagte gilt in gleicher Weise für acyliertes Guanidinobenzylamin.

Die Benennung der Reste P₂ und P₁ in der allgemeinen Formel I bezieht sich nicht auf die sonst üblicherweise verwendete Nomenklatur der Aminosäurereste in Peptidsubstraten von Serinproteasen und davon abgeleiteten Inhibitoren, wie sie von Schechter und Berger eingeführt wurde (Schechter und Berger, Biochem. Biophys. Res. Comm. 27, 157-162 (1967)). In allen Teilen der Erfindung, d.h. sowohl in der Beschreibung als auch in den Ansprüchen gelten die folgenden Definitionen:

Der Buchstabe P in Zusammenhang mit einer Zahl von 1 bis 3 in normaler Schrift, d.h. P1, P2 oder P3, wird für Aminosäurereste und deren Derivate entsprechend der Nomenklatur von Schechter und Berger verwendet. Dagegen steht der Buchstabe P in Zusammenhang mit einer tiefgestellten 1 oder 2, d.h. P₁ oder P₂, für Aminosäurereste und deren Derivate als Bestandteile der Formel I der vorliegenden Erfindung. Dabei entspricht die in der L-Konfiguration vorliegende substituierte oder unsubstituierte natürliche oder unatürliche Aminosäure P₁ in der Formel I P2 nach Schechter und Berger und die in der D-Konfiguration vorliegende substituierte oder unsubstituierte natürliche oder unatürliche Aminosäure P₂ in der Formel I entspricht P3 nach Schechter und Berger.

In Formel I ist
- R₂: ein H, ein verzweigter oder unverzweigter Alkylrest mit 1 bis 8 C-Atomen vorzugsweise mit 1 bis 3 C-Atomen, oder -(CH₂)_{c}COOR₈ mit c = 1, 2, 3 oder 4, wobei R₈ H oder ein verzweigter oder unverzweigter Alkylrest mit vorzugsweise 1 bis 6 C-Atomen, insbesondere 1 bis 3 C-Atomen, vor allem Ethyl, ist oder -(CH₂)_{d}-OR₉ mit d = 1, 2, 3 oder 4, wobei R₉ H ist, oder -(CH₂)ₑR₁₀, -(CH₂)ₑOR₁₀, -(CH₂)ₑSR₁₀, -(CH₂)ₑ-Guanidino, -(CH₂)ₑ-Imidazol oder -(CH₂)ₑNHR₁₀ mit e = 1, 2, 3, 4 oder 5 ist, wobei R₁₀ H, ein verzweigter oder unverzweigter Alkylrest mit 1-16, insbesondere 1-8, vor allem 1-3 C-Atomen oder ein substituierter oder unsubstituierter Aryl-, Heteroaryl-, Aralkyl- oder Heteroaralkylrest ist, wobei der Alkylrest vorzugsweise 1 bis 16, insbesondere 1 bis 8, vor allem 1 bis 3 C-Atome, und der Aryl- oder Heteroarylrest vorzugsweise 4 bis 14, insbesondere 6 bis 10, vor allem 6 C-Atome und vorzugsweise 1 bis 3 N als Heteroatom besitzt, oder -(CH₂)ₖO-CO-OR₁₆ mit k = 1, 2, 3, 4, 5, 6, 7 oder 8 ist, wobei R₁₆ ein verzweigtes oder unverzweigtes Alkyl mit 1-16, vorzugsweise 1-8, insbesondere 1-4, vor allem 1-2 C-Atomen, ein substituierter oder unsubstituierter Aryl-, Heteroaryl-, Aralkyl- oder Heteroaralkylrest, ein Adamantyl-, ein Campher-, ein Cyclohexylmethylrest, vorzugsweise Benzyl, ist; und
- R₅: -SO₂R₁₂ ist, wobei R₁₂ ein substituierter Aralkylrest, vorzugsweise Benzyl, ist;
wobei R₅ mit einer geladenen oder ungeladenen Gruppe, vorzugsweise einer -(CH₂)ⱼCOOR₁₃, -(CH₂)ⱼSO₂R₁₃, -(CH₂)ⱼNH₂, -(CH₂)ⱼ-Amidino-, -(CH₂)ⱼ-Hydroxyamidino- oder -(CH₂)ⱼ-Guanidino-Gruppe mit j = 0, 1 oder 2 modifiziert ist, wobei R₁₃ H oder ein Alkylrest mit vorzugsweise 1 bis 6 C-Atomen, insbesondere Ethyl, ist, oder
- R₂: -(CH₂)_{c}COOR₈ mit c = 1, 2, 3 oder 4, wobei R₈ H oder ein verzweigter oder unverzweigter Alkylrest mit vorzugsweise 1 bis 6 C-Atomen, insbesondere 1 bis 3 C-Atomen, vor allem Ethyl, ist oder -(CH₂)ₑSR₁₀, -(CH₂)ₑ-Guanidino, -(CH₂)ₑ-Imidazol oder -(CH₂)ₑNHR₁₀ mit e = 1, 2, 3, 4 oder 5 ist, wobei R₁₀ H, ein verzweigter oder unverzweigter Alkylrest mit 1-16, insbesondere 1-8, vor allem 1-3 C-Atomen oder ein substituierter oder unsubstituierter Aryl-, Heteroaryl-, Aralkyl- oder heteroaralkylrest ist, wobei der Alkylrest vorzugsweise 1 bis 16, insbesondere 1 bis 8, vor allem 1 bis 3 C-Atome, und der Aryl- oder Heteroarylrest vorzugsweise 4 bis 14, insbesondere 6 bis 10, vor allem 6 C-Atome und vorzugsweise 1 bis 3 N als Heteroatom besitzt, oder -(CH₂)ₖO-CO-OR₁₆ mit k = 1, 2, 3, 4, 5, 6, 7 oder 8 ist, wobei R₁₆ ein verzweigtes oder unverzweigtes Alkyl mit 1-16, vorzugsweise 1-8, insbesondere 1-4, vor allem 1-2 C-Atomen, ein substituierter oder unsubstituierter Aryl-, Heteroaryl-, Aralkyl- oder Heteroaralkylrest, ein Adamantyl-, ein Campher-, ein Cyclohexylmethylrest, vorzugsweise Benzyl, ist, und
- R₅: -SO₂R₁₂ ist; wobei R₁₂ ein substituierter oder unsubstituierter Aralkylrest, vorzugsweise Benzyl, ist;
wobei R₅ mit einer geladenen oder ungeladenen Gruppe, vorzugsweise einer -(CH₂)ⱼCOOR₁₃, -(CH₂)ⱼSO₂R₁₃, -(CH₂)ⱼNH₂, -(CH₂)ⱼ-Amidino-, -(CH₂)ⱼ-Hydroxyamidino- oder -(CH₂)ⱼ-Guanidino-Gruppe mit j=0, 1 oder 2 modifiziert sein kann, wobei R₁₃ H oder ein Alkylrest mit vorzugsweise 1 bis 6 C-Atomen, insbesondere Ethyl, ist;
und wobei P₁ in der Formel I in der L-Konfiguration vorliegt;
- R₄: -(CH₂)_{f}OR₁₁ mit f = 1 ist, wobei R₁₁ H ist und wobei P₂ in der Formel I in der D-Konfiguration vorliegt;
- U: ein Phenylrest ist;
- Z: in 3- oder 4-Position vorkommt und eine Aminomethyl-, eine Guanidino- oder eine Amidinogruppe 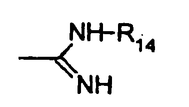
ist, wobei R₁₄ H, OH, NH₂, -COR₁₅ oder -COOR₁₅ ist, wobei R₁₅ ein verzweigter oder unverzweigter Alkylrest mit 1 bis 16, vorzugsweise 1 bis 8, insbesondere 1 bis 4, vor allem 1 bis 2 C-Atomen oder ein substituierter oder unsubstituierter Aryl- oder Heteroaryl-, Aralkyl- oder Heteroaralkylrest ist, wobei der Alkylrest vorzugsweise 1 bis 16, insbesondere 1 bis 8, vor allem 1 bis 4 und besonders bevorzugt 1 bis 2 C-Atome und der Aryl- oder Heteroarylrest vorzugsweise 4 bis 14, insbesondere 6 bis 10, vor allem 6 C-Atome und vorzugsweise 1 bis 3 N als Heteroatom besitzt;
**dadurch gekennzeichnet, dass** ein oder mehrere geladene Reste vorzugsweise abgeleitet von -COOH, -CH(COOH)₂, -SO₂H, NH₂, einer Amidino-, Hydroxyamidino-, Amidrazono- oder Guanidinogruppe in den Resten R₂ oder R₅ vorhanden sind;
oder eine Verbindung der allgemeinen Formel I in Form eines Prodrugs oder in Form ihres Salzes.

Ein Prodrug im Sinne der vorliegenden Erfindung ist ein acyliertes Amidino- oder Guanidinobenzylamin gemäß der allgemeinen Formel I, das als pharmazeutisch inaktives Derivat der entsprechenden pharmazeutisch wirksamen Substanz vorliegt und nach oraler Gabe spontan oder enzymatisch biotransformiert wird unter Freisetzung der pharmazeutisch wirksamen Substanz.

Weitere besonders bevorzugte Hemmstoffe der Urokinase, die besonders langsam eliminiert werden, sind 4-Amidinobenzylamin-Derivate gemäß der allgemeinen Formel I, wobei zusätzlich eine mit einer Amino- oder Carboxylgruppe funktionalisierte Oligo- oder Polyalkylenglycolkette, insbesondere eine Poly- oder Oligoethylenglycol- oder Poly- oder Oligopropylenglycolkette direkt an eine funktionelle Gruppe von R₂ insbesondere über eine -NH- oder eine -CO-Gruppe unter Ausbildung einer Amidbindung an R₂ gekoppelt ist, wobei die Oligo- oder Polyalkylenglycolkette zumindest an beiden Enden eine funktionelle Gruppe, insbesondere eine substituierte oder unsubstituierte Amino- und/oder Carboxylgruppe aufweist, oder wobei die Oligo- oder Polyalkylenglycolkette zumindest an einem Ende eine funktionelle Gruppe, insbesondere eine substituierte oder unsubstituierte Amino- und/oder Carboxylgruppe aufweist und am anderen Ende als Alkylether mit 1-4 C-Atomen, insbesondere als Methylether vorliegt, wobei R₂ vorzugsweise -(CH₂)ₙ-NH₂ mit n gleich 1-5, vorzugsweise 4 oder -(CH₂)ₙ-COOH mit n gleich 1-5, vorzugsweise 1-3 ist.

An eine Oligo- oder Polyalkylenglycolkette, die zumindest an beiden Enden eine funktionelle Gruppe, insbesondere eine substituierte oder unsubstituierte Amino- und/oder Carboxylgruppe aufweist, können zwei Moleküle der allgemeinen Formel I gekoppelt werden.

Werden die erfindungsgemäßen Derivate des 4-Amidinobenzylamins mit einer Oligo- oder Polyalkylenglykolkette gekoppelt, weist P1 in der allgemeinen Formel I vorzugsweise die folgende allgemeine Formel II auf: 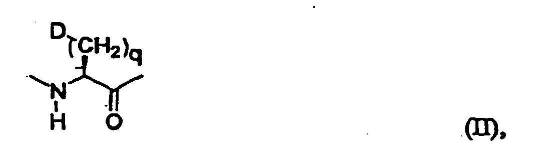 wobei q gleich 0, 1, 2, 3, 4 oder 5 ist und D gleich Formel III ist

E-F-G- (III),

wobei wenn E gleich eine H₂N-, HOOC-(CH₂)ₙ-CO-NH-, HOOC-, H₂N-(CH₂)ₙ-NH-CO- oder HS-Gruppe ist, F gleich ein Oligo- oder Polyalkylenglycol der allgemeinen Formel -(CH₂)_{d}-[O-CH₂-CH₂]ᵥO-(CH₂)ₘ-(NH-CO-CH₂-O-CH₂)ₖ- oder -(CH₂)_{d}-[O-CH(CH₃)-CF₂]ᵥ-O-(CH₂)ₘ-(NH-CO-CH₂-O-CH₂)ₖ- mit d =1, 2, 3 oder 4, v = eine ganze Zahl von 1 bis 1000, bevorzugt 2 bis 250, m = 0, 1, 2, 3 oder 4 und k = 0 oder 1 ist oder wenn E gleich eine CH₃-O-Gruppe ist, F gleich eine Oligo- oder Polyalkylenglycolkette der allgemeinen Formel -(CH₂)₄-[O-CH₂-CH₂]ᵥO-(CH₂)ₘ-(NH-CO-CH₂-O-CH₂)ₖ- oder -(CH₂)_{d}-[O-CH(CH₃)-CH₂]ᵥ-O-(CH₂)ₘ-(NH-CO-CH₂-O-CH₂)ₖ- mit d = 1, 2, 3 oder 4, v = eine ganze Zahl von 1 bis 1000, bevorzugt 1 bis 250, m = 0, 1, 2, 3 oder 4 und k = 0 oder 1 ist; und G gleich -CO-NH- oder -NH-CO- ist.

Ein besonderer Vorteil von Oligo- und/oder Polyalkylenglycolderivaten der erfindungsgemäßen Urokinasehemmstoffe besteht in ihrer verlängerten Halbwertszeit in der Zirkulation nach systemischer Gabe.

Weitere besonders geeignete Verbindungen sind Verbindungen nach den allgemeinen Formel I, wobei U an 1, 2 oder 3 Positionen vorzugsweise mit einem Halogen, insbesondere Fluor oder Chlor, oder einem Methyl-, Ethyl-, Propyl-, Methoxy-, Ethoxy-, oder Propoxyrest substituiert ist.

Weitere besonders geeignete Verbindungen sind Verbindungen nach den allgemeinen Formeln I, wobei eine Carboxylgruppe als Ester, bevorzugt als Ethylester, geschützt vorliegt

Weitere besonders geeignete Verbindungen sind Verbindungen nach den allgemeinen Formeln I oder II, wobei die Verbindung in Form eines Prodrugs vorliegt, wobei R₉ und/oder Ru in diesem Fall ein Alkylcarbonyl-, Aralkylcarbonyl-, Alkyloxycarbonyl- oder Aralkyrloxycarbonyl-Rest ist, wobei der lineare oder verzweigte Alkylrest vorzugsweise 1 bis 6, insbesondere 1 bis 4 C-Atome und der Arylrest vorzugsweise 5 bis 8, insbesondere 6 C-Atome besitzt.

Weitere besonders geeignete Verbindungen sind Verbindungen nach den allgemeinen Formeln I oder II, wobei im Amidinobenzylamidrest die Amidinogruppe in 4-Position steht und P₂ von der Aminosäure D-Ser abstammt und P₁ von Glycin, Alanin, Serin, Asparaginsäure oder Glutaminsäure abstammt und R₅ ein unsubstituierter oder mit einer Carboxylgruppe versehener Aryl- oder Aralkylsulfonyl-Rest mit 1 bis 16, vorzugsweise 1 bis 8, insbesondere 1 bis 4, vor allem 1 bis 2 C-Atomen im Alkylrest und 6 bis 14, vorzugsweise 6 bis 10, insbesondere 6 C-Atomen im Arylrest ist.

Weitere besonders geeignete Verbindungen sind Verbindungen nach den allgemeinen Formeln I oder II, wobei im Amidinobenzylamidrest die Amidinogruppe in 4-Position steht und P₂ die Aminosäure D-Ser und P₁ eine natürliche oder künstliche, unsubstituierte oder substituierte basische Aminosäure in der L-Konfiguration bedeutet, beispielsweise Lys, homoLys, Arg, norArg, homoArg, His, Orn, Orn(2-Imidazolinyl), Dab, 4-[(2-Amino)Pyrimidinyl]Buttersäure, Dap, Ala[3-(2-Pyrrolidinyl)], Ala[3-Pyrrolidinyl-(2-N-Amidino)], Ala[3-(N-Piperazine-4-N-amidino], Ala(4-Pip), Ala[4-Pip(N-amidino)], homoAla(4-Pip), Ala[3-Pip(N-amidino)], homoAla(3-Pip), homoAla[4-Pip(N-amidino)], Ala-(3-guanidino), Phe(3-Amidino), Phe(4-Amidino), Phe(3-NH₂), Phe(4-NH₂), Phe(3-Guanidino), Phe(4-Guanidino), Phe[4-(2-imidazolinyl)], Phe[3-CH₂-(guanidino)], Phe[4-CH₂-(guanidino)], homoPhe(3-Amidino), homoPhe(4-Amidino), hPhe(3-NH₂), hPhe(4-NH₂), hPhe(3-Guanidino), hPhe(4-Guanidino), cis-Cha(4-NH₂), trans-Cha(4-NH₂), cis-homoCha(4-NH₂), trans-homoCha(4-NH₂), trans-Cha(4-CH₂NH₂), trans-homoCha(4-CH₂NH₂), und wobei R₅ ein mit einer Sulfonylgruppe versehener Aryl- oder Aralkylsulfonyl-Rest mit 1 bis 16, vorzugsweise 1 bis 8, insbesondere 1 bis 4, vor allem 1 bis 2 C-Atomen im Alkylrest und 6 bis 14, vorzugsweise 6 bis 10, insbesondere 6 C-Atomen im Arylrest ist, der an die Aminogruppe des D-Ser gebunden ist, wobei P₁ ganz besonders bevorzugt die Aminosäure Lysin oder Arginin ist.

Weitere besonders geeignete Verbindungen sind Verbindungen nach den allgemeinen Formeln I oder II, wobei der Substituent am substituierten Aryl-, Heteroaryl-, Aralkyl- oder Heteroaralkylrest ein Halogen, vorzugsweise Fluor, Chlor oder Brom, insbesondere Fluor oder Chlor, ist.

Weitere besonders geeignete Verbindungen sind Verbindungen nach den allgemeinen Formeln I oder II, wobei eine Verbindung der allgemeinen Formel I die folgende Struktur aufweist: 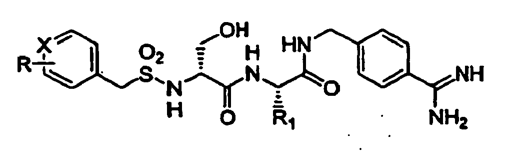 mit R gleich COOH oder COOMe in ortho-, meta- oder para- oder H und X gleich CH und R₁ gleich H; oder
R gleich 4-COOH oder 3-COOH mit X gleich CH und R₁ gleich H, CH₃ oder CH₂-OH; oder
R gleich 4-CN mit X gleich CH und R₁ gleich CH₃; oder
R gleich 4-(NH₂-CH₂) mit X gleich CH und R₁ gleich H; oder
R gleich H mit X gleich CH und R₁ gleich H, CH₂-OH, CH₂-O(Bzl), CH₂-NH₂, CH(OH)CH₃ oder CH(OHzl)CH₃; oder
R gleich 4-COOMe mit X gleich CH und R₁ gleich CH₂-OH; oder
R gleich 4-Cl, 4-Me, 4-F oder 3,4-Di-Cl mit X gleich CH und R₁, gleich H; oder R gleich H mit X gleich N und R₁ gleich H.

Weitere besonders geeignete Verbindungen sind Verbindungen nach den allgemeinen Formeln I oder II, wobei eine Verbindung der allgemeinen Formel I eine der folgenden Strukturen aufweist: 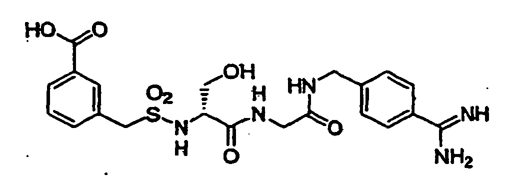 oder 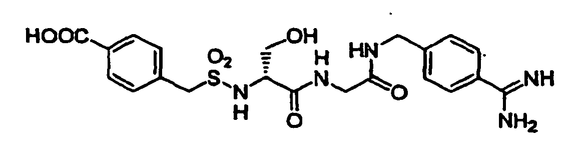 oder

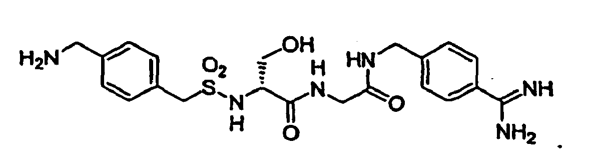

Weitere besonders geeignete Verbindungen sind Verbindungen nach den allgemeinen Formeln I oder II, wobei eine Verbindung der allgemeinen Formel I oder II eine der folgenden Strukturen aufweist: 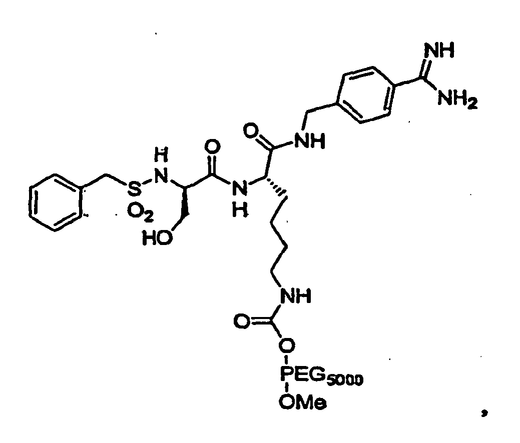 wobei PEG₅₀₀₀ eine Polyethylenglyrolkette mit einem durchschnittlichen Molekulargewicht von 5000 Da bedeutet, wobei ebenso Polyethylenglykolketten mit einem durchschnittlichen Molekulargewicht von 100 - 20000 Da verwendet werden können;
oder 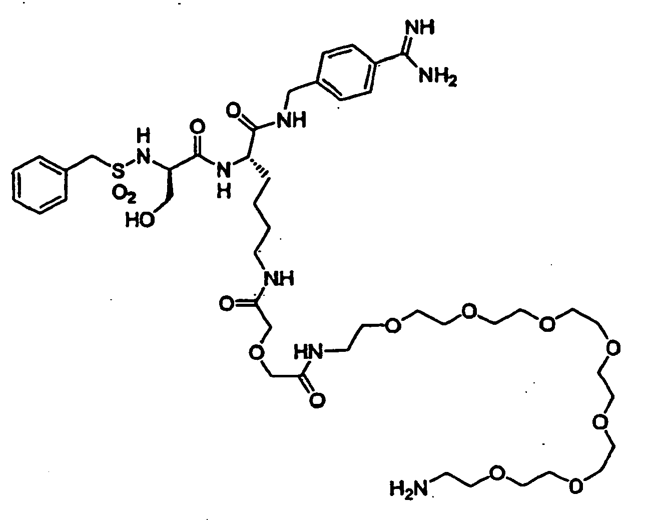 oder 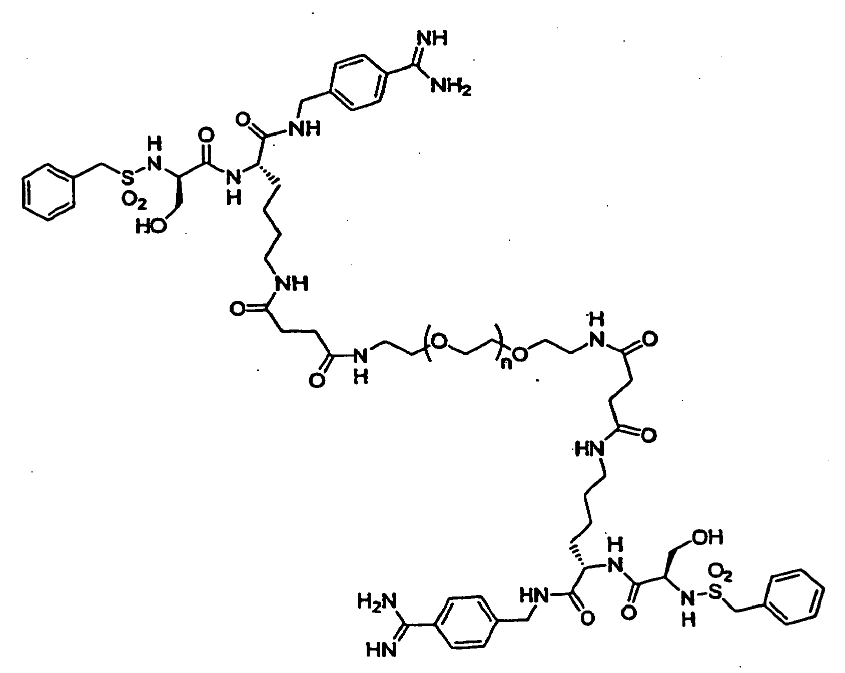 mit n = 2 bis 250.

Bei einer starken Inaktivierung von Urokinase werden die zusätzlich geladenen 4-Amidinobenzylanlin-Derivate auf vorteilhafte und überraschende Weise sehr langsam eliminiert, so dass die erfindungsgemäßen Verbindungen eine neue Gruppe von hochaktiven Urokinase-Hemmstoffen darstellen.

Beispiele für solche Verbindungen sind neben den in den Ausführungsbeispielen genannten:
(3-Pyridylamethyl)sulfonyl-dSer-Gly-4-Amidinobenzylamid
(3-Pyridylmethyl)sulfonyl-dSer-Ala-4-Amidinobenzylamid
(3-Pyridylmethyl)sulfonyl-dSer-Ser-4-Amidinobenzylamid
(3-Pyridyhnethyl)sulfonyl-dSer-Pm-4-Amidinobenzylamid

(4-Pyridylmethyl)sulfonyl-dSer-Ala-4-Amidinobenzylamid
(4-Pyridylmethyl)sulfonyl-dSer-Ser-4-Amidinobenzylamid
(4-Pyridylmethyl)sulfonyl-dSer-Pro-4-Amidinobenzylamid

(2-Pyridylmethyl)sulfonyl-dSer-Gly-4-Amidinobenzylamid
(2-Pyridylmethyl)sulfonyl-dSer-Ala-4-Amidinobenzylamid
(2-Pyridylmethyl)sulfonyl-dSer-Ser-4-Amidinobenzylamid
(2-Pyridylmethyl)sulfonyl-dSer-Pro-4-Amidinobenzylamid

Einen besonders bevorzugten Hemmstoff von Urokinase mit hoher Affinität, der ebenfalls besonders langsam eliminiert wird, bildet acyliertes 4-Amidinobenzylamin, das als P₁ (P2) Aminosäure eine natürliche oder künstliche, unsubstituierte oder substituierte basische Aminosäure in der L-Konfiguration, besonderes bevorzugt Arginin oder Lysin besitzt, wenn D-Serin als P₂ (P3) Rest gebunden ist, und wenn die Verbindung eine N-terminale Schutzgruppe R₅ aus einem Aryl- bzw. Aralkyl-sufonyl-Rest aufweist.

Bei einer starken Inaktivierung von Urokinase werden die zusätzlich geladenen 4-Amidinobenzylamin-Derivate auf vorteilhafte und überraschende Weise sehr langsam eliminiert, so dass die erfindungsgemäßen Verbindungen eine neue Gruppe von hochaktiven Urokinase-Hemmstoffen darstellen.

Beispiele für solche Verbindungen sind neben den bereits genannten:
Benzylsulfonyl-dSer-homoLys-4-Amidinobenzylamid
Benzylsulfonyl-dSer-norArg-4-Amidinobenzylamid
Benzylsulfonyl-dSer-homoArg-4-Amidinobenzylamid
Benzylsulfonyl-dSer-Orn-4-Amidinobenzylamid
Benzylsulfonyl-dSer-Orn(2-Imidazolinyl)-4-Amidinobenzylamid
Benzylsulfonyl-dSer-His-4-Amidinebenzylamid
Benzylsulfonyl-dSer-Dab-4-Amidinobenzylamid
Benzylsulfonyl-dSer-4-[(2-Amino)Pyrimidinyl]Buttersäure-4-Amidinobenzylamid
Benzylsulfonyl-dSer-Dap-4-Amidinobenzylamid
Benzylsulfonyl-dSer-Ala[3-(2-Pyrrolidinyl)]-4-Amidinobenzylamid
Benzylsulfonyl-dSer-Ala[3-Pyrrolidinyl-(2-N-Amidino)]-4-Amidinobenzylamid
Benzylsulfonyl-dSer-Ala[3-(N-Piperazine-4-N-amidino]-4-Amidinobenzylamid
Benzylsulfonyl-dSer-Ala(4-Pip)-4-Amidinobenzylamid
Benzylsulfonyl-dSer-Ala[4-Pip(N-amidino)]-4-Amidinobenzylamid
Benzylsulfonyl-dSer-homoAla(4-Pip)-4-Amidinobenzylamid
Benzylsulfonyl-dSer-Ala[3-Pip(N-amidino)]4-Amidinobenzylamid
Benzylsulfonyl-dSer-homoAla(3-Pip)-4-Amidinobenzylamid
Benzylsulfonyl-dSer-homoAla[4-Pip(N-amidino)]-4-Amidinobenzylamid
Benzylsulfonyl-dSer-Ala-(3-guanidino)-4-Amidinobenzylamid
Benzylsulfonyl-dSer-Phe(3-Amidino)-4-Amidinobenzylamid
Benzylsulfonyl-dSer-Phe(4-Amidino)-4-Amidinobenzylamid
Benzylsulfonyl-dSer-Phe(3-NH₂)-4-Amidinobenzylamid
Benzylsulfonyl-dSer-Phe(4-NH₂)-4-Amidinobenzylamid
Benzylsulfonyl-dSer-Phe(3-Guanidino)-4-Amidinobenzylamid
Benzylsulfonyl-dSer-Phe(4-Guanidino)-4-Amidinobenzylamid
Benzylsulfonyl-dSer-Phe[4-(2-imidazolinyl)]-4-Amidinobenzylamid
Beazylsulfonyl-dSer-Phe[3-CH₂-(guanidino)]-4-Amidinobenzylamid
Benzylsulfonyl-dSer-Phe[4-CH₂-(guanidino)]-4-Amidinobenzylamid
Benzylsulfonyl-dSer-homoPhe(3-Amidino)-4-Amidinobenzylamid
Benzylsulfonyl-dSer-homoPhe(4-Amidino)-4-Amidinobenzylamid
Benzylsulfonyl-dSer-hPhe(3-NH₂)-4-Amidinobenzylamid
Beazylsulfonyl-dSer-hPhe(4-NH₂)-4-Amidinobenzylamid
Benzylsulfonyl-dSer-hPhe(3-Guanidino)-4-Amidinobenzylamid
Benzylsulfonyl-dSer-hPhe(4-Guanidino)-4-Amidinobenzylamid

Benzylsulfonyl-dSer-cis-Cha(4-NH₂)-4-Amidinobenzylamid
Benzylsulfonyl-dSer-trans-Cha(4-NH₂)-4-Amidinobenzylamid
Benzylsulfonyl-dSer-cis-homoCha(4-NH₂)-4-Amidinobenzylamid
Benzylsulfonyl-dSer-trans-homoCha(4-NH₂)-4-Amidinobenzylamid
Benzylsulfonyl-dSer-trans-Cha(4-CH₂NH₂)4-Amidinobenzylamid
Benzylsulfonyl-dSer-trans-homoCha(4-CH₂NH₂)-4-Amidinobenzylamid

Die Verbindungen liegen in der Regel als Salze, vorzugsweise mit Mineralsäuren, bevorzugt als Hydrochloride, vor oder vorzugsweise als Salze mit geeigneten organischen Säuren. Bevorzugte Salze von Mineralsäuren sind auch Sulfate. Geeignete organische Säuren sind beispielsweise Essigsäure, Ameisensäure, Methylsulfonsäure, Bernsteinsäure, Apfelsäure oder Trifluoressigsäure, wobei bevorzugte Salze von organischen Säuren Acetate sind.

Die Verbindungen der allgemeinen Formel I können in prinzipiell bekannter Weise, wie nachfolgend beschrieben, beispielsweise wie folgt hergestellt werden:

Aus dem kommerziell erhältlichen 4-Cyanobenzylamin (Showa Denko, Japan) wird das Boc-geschützte 4-Acetyloxamidinobenzylamin nach dem Fachmann bekannten Verfahren gewonnen (Judkins et aL, Synth. Commun. 26, 4351 (1996)). Nach Abspaltung der Boc-Schutzgruppe erfolgt die Ankopplung der weiteren Aminosäuren und der Schutzgruppe R₅ mittels Standardkopplungsmethoden mit Boc als N-terminaler Schutzgruppe. Die P₂ (P3) Aminosäure kann auch direkt als N-aryl- bzw. N-aralkyl-sulfonyl-geschützte Aminosäure gekoppelt werden. Die Peptidanaloga werden sequentiell, beginnend vom Acetyloxamidinobenzylamin, aufgebaut. Die meisten Zwischenprodukte kristallisieren gut und lassen sich damit einfach reinigen. Die Endreinigung der Hemmstoffe erfolgt auf der letzten Stufe vorzugsweise über präparative, reversed-phase HPLC.

Gegenstand der vorliegenden Erfindung ist auch ein Verfahren zur Herstellung einer Verbindung der allgemeinen Formel I oder II, wobei sequentiell die entsprechenden Aminosäuren an ein 4-Acetyloxamidinobenzylamin angekoppelt werden, wobei die N-terminale Aminosäure entweder den R₅-Rest bereits trägt oder dieser anschließend daran gebunden wird.

Ein weiterer Gegenstand der Erfindung ist ein Arzneimittel enthaltend einen erfindungsgemäßen Hemmstoff sowie weitere pharmazeutisch geeignete Hilfs- und/oder Zusatzstoffe. Geeignete Hilfs- und/oder Zusatzstoffe, die z.B. der Stabilisierung und/oder Konservierung des Arzneimittels dienen, sind dem Fachmann allgemein geläufig (z.B. Sucker H. et al., (1991) Pharmazeutische Technologie, 2. Auflage, Georg Thieme Verlag, Stuttgart). Hierzu zählen beispielsweise physiologische Kochsalzlösungen, Ringer-Dextrose, Ringer-Laktat, entmineralisiertes Wasser, Stabilisatoren, Antioxidantien, Komplexbildner, antimikrobielle Verbindungen, Proteinaseinhibitoren und/oder inerte Gase.

Das Arzneimittel könnte beispielsweise in parenteraler Anwendungsform, insbesondere in intraartieller, intravenöser, intramuskulärer oder subcutaner Form, in enteraler Anwendungsform, insbesondere zur oralen oder rektalen Anwendung oder in topischer Anwendungsform, insbesondere als Dermatikum, angewendet werden. Bevorzugt sind intravenöse oder subkutane Anwendungen.

In einer Ausführungsform der Erfindung wird das Arzneimittel beispielsweise in Form einer Tablette, eines Dragées, einer Kapsel, eines Pellets, Suppositoriums, einer Lösung, insbesondere einer Injektions- oder Infusionslösung, von Augen-, Nasen und Ohrentropfen, eines Safts, einer Emulsion oder Suspension, eines Globuli, Styli, Aerosols, Puders, einer Paste, Creme oder Salbe eingesetzt.

Die erfindungsgemäßen Urokinasehemmstoffe oder die genannten Arzneimittel werden bevorzugt zur Diagnose, Therapie oder Prophylaxe eines Tumors, insbesondere der Reduzierung der Bildung von Tumormetastasen, bevorzugt in oraler, subkutaner, intravenöser oder transdermaler Form verwendet.

Die Erfindung soll nachstehend anhand von 14 Ausführungsbeispielen näher erläutert werden, ohne sie zu beschränken, wobei Vergleichsbeispiel 5 den allgemeinen Hintergrund der Erfindung betrifft.

### Methoden

Analytische HPLC: Shimadzu LC-10A System, Säule: Vydac C₁₈, 5 µm (250 x 4 mm) Lösungsmittel A: 0,1 % TFA in Wasser, B: 0,1 % TFA in ACN, Gradient: 10 % B bis 60 % B in 50 min, 1 ml/min Fluß, Detektion bei 220 oder 215 nm.

Präparative HPLC: Shimadzu LC-8A System, Säule: Knauer C₁₈, 5 µm (250 x 32 mm) Lösungsmittel A: 0,1 % TFA in Wasser, B: 0,1 % TFA in ACN, Gradient: 10 % B bis 55 % B in 120 min, 10 ml/min Fluß, Detektion bei 220 nm.

Massenspektroskopie: Die Massenspektren wurden auf einem Kompact Probe der Firma Kratos (Manchester, England) mit einem Flugzeitmessungsdetektor und α-Cyano-Hydroxyzimtsäure als Matrix, bzw. auf einem ESI-MS LCQ der Firma Finnigan (Bremen, Deutschland), gemessen.

### Beispiel 1: Synthese von Benzylsulfonyl-D-Ser-Glu-4-Amidinobenzylamid x TFA

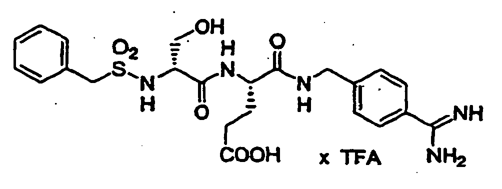

### 1a) Boc-4-Cyanobenzylamid

20 g (0,151 mol) 4-Cyano-benzylamin wurden in 300 ml H₂O, 150 ml Dioxan und 150ml 1N NaOH gelost. Unter Eiskühlung wurden 37,5 ml Di-tert-butyldicarbonat zugetropft und eine Stunde bei 0°C sowie weitere 24 Std. bei Raumtemperatur gerührt. Das Dioxan wurde im i.V. entfernt und das Produnkt wurde in Essigester und 5 % KHSO₄-Lösung aufgenommen. Die Essigesterphase wurde 3Mal mit 5%-iger KHSO₄- und 3-mal mit gesättigter NaCl-Lösung gewaschen, über Na₂O₄ getrocknet und i.V. eingeengt (weiBe Kristalle). HPLC: Acetonitril/H₂O, Elution bei 44,1 % Acetonitril; Ausbeute: 30,48 g (0,131 mol), 87 %.

### 1b) Boc-4-Acetyloxamidinobeazylamid

Nach Judkins et al. (Synthetic Comm. 26, 4351-4367, 1996) wurden 30,48 g (0,131 mol) Boc-4-Cyanobenzylamid mit 13,65 g (0,197 mol) Hydroxylamin x HCl und 34 ml (0,197 mol) DIEA in 300 ml abs. Ethanol gelöst Es wurde 2 Std. unter Rückfluss gekocht und über Nacht bei Raumtemperatur gerührt. Danach wurde der Ansatz i.V. eingeengt, der Rückstand in ca. 200 ml Essigsäure gelöst und mit 18,67ml (0,197 mol) Essigsäureaahydrid versetzt. Nach 1 Std. wurde erneut eingeengt, in Essigester gelöst und bei 0 °C je 3-mal mit 5 %iger KHSO₄- und gesättigter NaCl-Lösung gewaschen. Nach dem Trocknen über Na₂SO₄ und Einengen i.V. fiel ein weiBes Pulver an. HPLC: Acetonitril/H₂O, Elution bei 32,0 % Acetonitril; Ausbeute: 31,3 g (0,102 mol) 78 %.

### 1c) 4-Acetyloxamidinobenzylamin x HCl

5 mmol Boc-4-Acetyloxamidinobenzylamid werden in 20 ml 1 N HCl in Eisessig gelöst und 45 min bei Raumtemperatur stehen gelassen. Dann wird i.V. weitgehend eingeengt, das Produkt mit trockenem Diethylether gefällt, abgefrittet, nochmals mit frischem Ether gewaschen und i.V. getrocknet. Auf Grund der quantitativen Umsetzung wurde das Produkt ohne weitere Reinigung für den nächsten Syntheseschritt eingesetzt.

### 1d) Boc-Glu(OBzl)-4-Acetyloxamidinobenzylamid

Die Kopplung von Boc-Glu(OBzl)-OH (Orpegen, Heidelberg) an 4-Acetyloxamidinobenzylamin x HCl erfolgte nach Frérot et al. (Tetrahedron 47, 259 ff., 1991). Dazu wurden 2,27 g (9,3 mmol) 4-Acetyloxamidinobenzylamin x HCl und 3,138 g (9,3 mmol) Boc-Glu(OBzl)-OH in ca. 25 ml DMF gelost. Bei 0°C wurden 4,84 g (9,3 mmol) PyBOP und 3,878 ml (27,9 mmol) TEA zugegeben und der pH-Wert mit TEA auf 9 eingestellt. Nach 1 Std. Rühren bei Raumtemperatur wurde i.V. eingeengt, in Essigester aufgenommen und je 3-mal saner, basisch und neutral gewaschen, mit Na₂SO₄ getrocknet und i.V. eingeengt. Ausbeute: 4,1 g (7,8 mmol) 84 %.

### 1e) H-Glu(OBzl)-4-Acetyloxamidinobenzylamid x HCl

4,1 g Boc-Glu(Bzl)**-**4-Acetyloxamidinobenzylamid wurden in 100 ml 1 N HCl in Eisessig gelöst und 45 min bei Raumtemperatur stehen gelassen. Dann wurde i.V. weitgehend eingeengt und mit trockenem Diethylether gefällt, danach abgefrittet und das Produkt nochmals mit frischem Ether gewaschen. Nach Trocknen des Produkts i.V. wurde es ohne weitere Aufreinigung für die Synthese nach Punkt 1g) eingesetzt.

### 1f) Benzylsulfonyl-D-Ser(Bzl)-OH

229 mg (1,173 mmol) H-D-Ser(Bzl)-OH und 408 µl (2,345 mmol) DIEA wurden in 50 ml 50 % Acetonitril gelöst. Dann wurden 335 mg (1,76 mmol) Benzylsulfonylchlorid zugegeben und 12 Std. bei Raumtemperatur gerührt. Es wurde i.V. eingeengt, mit Essigester aufgenommen und je 3-mal sauer und neutral gewaschen. Nach Trocknen über Natriumsulfat wurde i.V. eingeengt. Ausbeute: 289 mg (0,827 mmol) 71 %.

### 1g) Benzylsnlfonyl-D-Ser(Bzl)-Glu(OBzl)-4-Acetyloxamidinobenzylamid

151 mg (0,433 mmol) Benzylsulfonyl-D-Ser(Bzl)-OH und 194 mg (0,433 mmol). H-Glu(OBzl)-4-Acetyloxamidinobenzylamid x HCl wurden in 5 ml abs. DMF gelöst. Unter Eiskühlung wurden 225 mg (0,433 mmol) PyBOP und 230 µl (1,32 mmol) DIEA zugegeben. Nach 2 Std. wurde i.V. eingeengt, mit Essigester aufgenommen und je 3-mal sauer, basisch und neutral gewaschen. Nach Trocknen über Natriumsulfit wurde i.V. eingeengt und ohne weitere Aufarbeitung nach Punkt 1.8 hydriert. Ausbeute: 270 mg (0,364 mmol) 84 %.

### 1h) Benzylsulfonyl-D-Ser-Glu-4-Amidinobenzylamid x TFA

270 mg (0,364 mmol) Bzls-D-Ser(Bzl)-Glu(OBzl)-4-Acetyloxamidinobenzylamid wurden in 30 ml 90 %iger Essigsäure gelöst Anschließend wurden unter Argon 20 mg 10 % Palladium auf Aktivkohle zugesetzt. Argon wurde durch eine Wasserstofftmosphäre ersetzt und der Ansatz unter kräftigem Rühren 24 Std. hydriert. Der Katalysator wurde abfiltriert, das Filtrat iV. eingeengt und das Produkt mit präparativer reversed-phase HPLC gereinigt (Acetonitril/H₂O, 0,1 % Trifluoressigsäure, Elution bei 22,6 % Acetonitril).

### Beispiel 2: Hemmung von Urokinase durch ausgewählte 4-Amidinobenzylamid-Verbindungen

**Tabelle 1**

| | Konfiguration | | | | | Kᵢ, µM |
|---|---|---|---|---|---|---|
| R₅ | R₄ | R₄ | R₃ | X-R₂ | Y-R₁ | |
| Bzl-SO₂ | D | CH₂-OH | H | CH₂ | CH₂ | 0,036 |
| Bzl-SO₂ | D | CH₂-OH | H | CH-CH₃ | CH₂ | 0,0077 |
| Bzl-SO₂ | D | CH₂-OH | H | CH-CH₂-COOH | CH₂ | 0,86 |
| Bzl-SO₂ | D | CH₂-OH | H | CH-(CH₂-)₂-COOH | CH₂ | 0,16 |

### Bestimmung der Hemmwirkung

Zur Bestimmung der Hemmwirkung wurden 200 µl Tris-Puffer (0,05 M, 0,154 M NaCl 5 % Ethanol, pH 8,0; enthält den Inhibitor), 25 µl Substrat (Bzl-βAla-Gly-Arg-pNA in H₂O) und 50 µl sc-Urokinase bei 25 °C inkubiert. Nach 3 min wurde die Reaktion durch Zugabe von 25 µl Essigsäure (50%) unterbrochen und die Absorption bei 405 nm mittels Microplate Reader (Dynatech MR 5000) bestimmt. Die Kᵢ-Werte wurden nach Dixon (Biochem. J. 55, 170-171, 1953) durch lineare Regression mittels eines Computerprogramms ermittelt. Die Kᵢ-Werte sind das Mittel aus mindestens drei Bestimmungen.

### Beispiel 3: Elimination nach i.v.-Gabe von 1 mg/kg Körpergewicht an der Ratte von Derivaten des Benzylsulfonyl-D-Ser-Gly-4-amidinobenzylamids mit Ala bzw. Glu in P2-Position

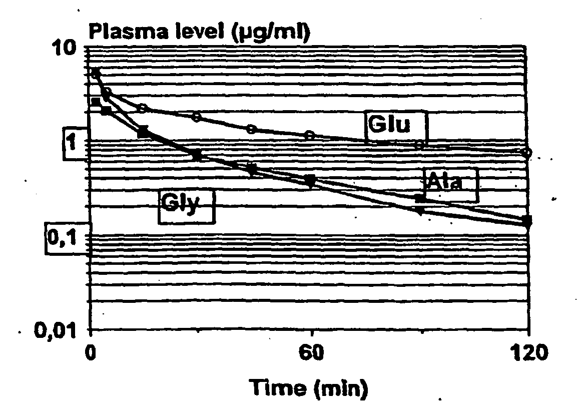

### Tierversuche

Weibliche Wistar Ratten (240-300 g Körpergewicht) wurden narkotisiert (Ethylurethan 2,5 g/ml in NaCl, 0,5 ml/100 g Ratte), anschließend erfolgte die Präparation der am Hals gelegenen *A. carotis*. Ein in diesem Gefäß fixierter Katheter ermöglichte die Blutentnahme zu festgelegten Zeiten. Das Applikationsvolumen betrug 0,5 ml, als Applikationslösung wurde 0,9% NaCl eingesetzt. Blutproben à 500 µl (versetzt im Verhältnis 19 + 1 mit 1,04 M Natriumcitrat) wurden zu folgenden Zeitpunkten entnommen**:** 2, 5, 15. 30, 45, 60, 90, 120, 150, 180, 210, 240 und 270 min. Der entstandene Blutverlust wurde unmittelbar nach Entnahme der Probe mit 500 µl 0,9 % NaCl-Lösung kompensiert. Citratplasma wurde durch Zentrifugation des Blutes bei 1200*g, für 10 min erhalten. Die Konzentration der Wirkstoffe im Plasma wurde mittels HPLC ermittelt.

### Beispiel 4: 3-(HOOC)Benzylsulfonyl-dSer-Gly-4-Amidinobenzylamid x TFA

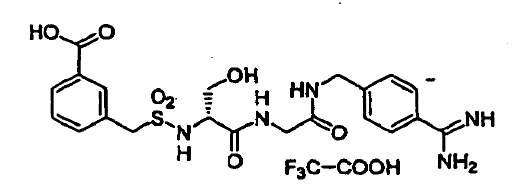

### 4a) 3-(COOMe)-Benzylsulfonsäure Natrium-Salz

5 g (21,1 mmol) 3-(Bromomethyl)Benzoesäuremethylester (Lancaster) wurden in 35 ml Wasser suspendiert und nach Zugabe von 2,94 g (23,3 mmol) Na₂SO₃ 8 h unter Rückfluss gekocht. Der Ansatz wurde heiß filtriert und das Wasser im Vakuum bis zur beginnenden Kristallisation eingeengt. Der Ansatz wurde über Nacht im Kühlschrank aufbewahrt, danach wurden die Kristalle abgesaugt und nochmals aus Wasser umkristallisiert. Die Kristalle wurden abgesaugt und im Vakuum getrocknet.
Ausbeute: 3.9 g (15,46 mmol) HPLC: 22,3 % B

### 4b) 3-(COOMe)-Benzylsulfonylchlorid

2,5 g (9,91 mmol) 3-(COOMe)-Benzylsulfonsäure Natrium-Salz wurden mit ca. 10 ml Phosphorylchlorid angefeuchtet, mit 2,27 g (10,9 mmol) PCl₅ versetzt und 15 Minuten im Eisbad gerührt. Danach wurde der Absatz 4 h auf 80 °C erwärmt. Anschließend wurde der Ansatz auf Eis gegossen und 30 min kräftig gerührt, das Produkt schied sich in Form weißer Kristalle auf dem Eis ab. Nachdem das Eis teilweise aufgetant war, wurde der Ansatz durch eine Fritte filtriert und das verbleibende Produkt-Eis-Gemisch mehrmals mit Wasser gewaschen. Die verbleibenden Kristalle wurden im Vakuum getrocknet.
Ausbeute: 1,6 g (6,43 mmol) 65 % (weiße Kristalle)

### 4c) 3-(COOMe)-Benzylsulfonyl-dSer(tBu)-OH

0,75 g (4,65 mmol) H-dSer(tBu)-OH (Bachem) wurden in 60 ml trockenem DCM suspendiert, mit 1,23 ml (9,765 mmol) Trimethylsilylchlorid und 1,7 ml (9,765 mmol) DIEA versetzt. Der Ansatz wurde 1,0 h unter Rückfluss gekocht und danach im Eisbad abgekühlt. Anschließend wurden 1,27 g (5,12 mmol) 3-(COOMe)-Benzylsulfonylchlorid und 1,04 ml (6 mmol) DIEA in mehreren Portion innerhalb von 30 min zugegeben. Der Ansatz wurde weitere 15 min unter Eiskühlung und danach für 3 h bei Raumtemperatur gerührt. Das Lösungsmittel wurde im Vakuum entfernt, der Rückstand in Wasser (mit 1 N NaOH auf pH 8,5-9 gebracht) gelöst und 2 x mit Ether extrahiert. Die wässrige Phase wurde mit 5 % KHSO₄-Lösung angesäuert und 3 x mit Essigester extrahiert. Die vereinte Essigesterphase wurde jeweils 3 x mit 5 % KHSO₄-Lösung und NaCl-gesättigter Lösung gewaschen und mit Na₂SO getrocknet. Anschließend wurde das Lösungsmittel im Vakuum entfernt.
Ausbeute: 1,3 g (3,48 mmol Feststoff), HPLC: 51 % B

### 4d) H-Gly-4-Acetyloxamidinobenzylamid x HCl

2 g (5,49 mmol) Boc-Gly-4-Acetyloxamidinobenzylamid (hergestellt wie in WO 01/96286 A2 beschrieben) wurden mit 30 ml 1 N HCl in Eisessig versetzt. Der Ansatz wurde gelegentlich umgeschüttelt. Nach 45 min wurde das Lösungsmittel etwas eingeengt und das Produkt durch Zugabe von Diethylether gefällt, auf einer Fritte abgesaugt, mit Ether gewaschen und im Vakuum getrocknet.
Ausbeute: 1,55 g (5,15 mmol), weißer Feststoff

### 4e) 3-(COOMe)-Benzylsulfonyl-dSer(tBu)-Gly-Acetyloxamidinobenzylamid

1 g (2,68 mmol) 3-(COOMe)-Benzylsulfonyl-dSer(tBu)-OH und 0,84 g (2,8 mmol) H-Gly-4-Acetyloxamidinobenzylamid x HCl wurden unter Rühren und Eiskühlung in 15 ml DMF gelöst und mit 1,39 g (2,68 mmol) PyBop sowie 1,26 ml (7,236 mmol) DIEA versetzt. Nach 30 min wurde das Eisbad entfernt und weitere 4 h bei Raumtemperatur gerührt. Das DMF wurde im Vakuum eingeengt, der verbleibende Rückstand in Essigester gelöst und jeweils 3 x mit 5 % KHSO₄, NaCl-gesättigtem Wasser, gesättigter NaHCO₃-Lösung und nochmals mit NaCl-gesättigtem Wasser gewaschen. Die Essigesterphase wurde mit Na₂SO₄ getrocknet und anschließend das Lösungsmittel im Vakuum entfernt. Das Rohprodukt wurde ohne weitere Reinigung für den nächsten Syntheseschritt verwendet.
Ausbeute: 1,35 g (2,18 mmol) Öl, HPLC: 47,89 % B

### 4f) 3-(COOMe)-Benzylsulfonyl-dSer(tBu)-Gly-4-Amidinobenzylamid x Acetat

1 g (1,61 mmol ) 3-(COOMe)-Benzylsulfonyl-dSer(tBu)-Gly-4-Acetyloxamidinobenzylamid wurden in 65 ml 90 % Essigsäure gelöst, mit 150 mg Katalysator (10 % Pd auf Aktivkohle) versetzt und über Nacht mit Wasserstoff hydriert. Der Katalysator wurde abfiltriert und das Lösungsmittel im Vakuum eingeengt. Der verbleibende Rückstand wurde mit Toluen versetzt, danach wurde das Lösungsmittel im Vakuum wieder entfernt. Dieser Vorgang wurde nochmals wiederholt. Der verbleibende Rückstand wurde direkt für den nächsten Reaktionsschritt verwendet.
Ausbeute: 0,9 g (1,44 mmol) Feststoff, HPLC: 39,75 % B
Ca. 50 mg des Rohproduktes wurden mit präparativer reversed-phase HPLC gereinigt und lyophilisiert.
MS: berechnet 561,2 (monoisotopic), gefunden 562,9 [M+H]⁺

### 4g) 3-(COOH)-Benzylsulfonyl-dSer(tBu)-Gly-4-Amidinobenzylamid x TFA

750 mg (1,2 mmol) 3-(COOMe)-Benzylsulfonyl-dSer(tBu)-Gly-4-Amidinobenzylamid x Acetat wurden in 20 ml Methanol und 10 ml Wasser gelöst und mit 4 ml 1 N LiOH versetzt. Der Ansatz wurde über Nacht gerührt, nach ca. 15 h mit 5 % KHSO₄ neutralisiert (pH 6-7) und das Lösungsmittel im Vakuum entfernt. Das Rohprodukt wurde mittels präparativer reversed-phase HPLC gereinigt und lyophilisiert.
HPLC: 34,16 % B (weißer Feststoff)

### 4h) 3-(COOH)-Beazylsulfonyl-dSer-Gly-4-Amidinobenzylamid

100 mg (0,151 mmol) 3-(COOH)-Benzylsulfonyl-dSer(tBu)-Gly-4-Amidinobenzylamid wurden mit 0,5 ml Wasser und 4,5 ml Trifluoressigsäure versetzt. Der Ansatz wurde 60 min bei Raumtemperatur belassen und danach das Lösungsmittel im Vakuum eingeengt. Der Rückstand wurde in Wasser gelöst und lyophilisiert.
Ausbeute: 91 mg (weißer Feststoff) HPLC: 23,47 % B

### Vergleichsbeispiel 5: Benzylsulfonyl-dSer-Ser-4-Amidinobenzylamid x TFA

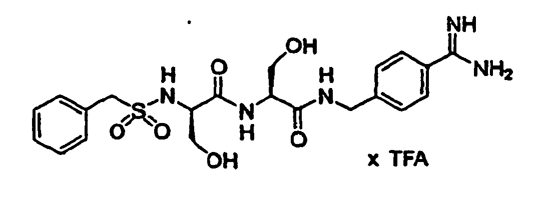

### 5a) Boc-Ser(Bzl)-4-Acetyloxamidinobenzylamid

4,847 g (16,41 mmol) Boc-Ser(Bzl)-OH wurden in 50 ml THF gelöst und bei -15 °C mit 1,805 ml (16,41 mmol) NMM und 2,133 ml CKBIE versetzt. Der Ansatz wurde 10 min bei -15 °C gerührt, dann wurden 4 g (16,41 mmol) 4-(Acetyloxamidino)Benzylamin x HCl (hergestellt wie in WO 01/96286 A2 beschrieben) und nochmals 1,805 ml (16,41 mmol) NMM hinzugefügt. Der Ansatz wurde eine weitere Stunde bei -15 °C und über Nacht bei Raumtemperatur gerührt. Das Lösungsmittel wurde im Vakuum entfernt, der Ansatz in Essigester aufgenommen und jeweils 3 x mit 5 % KHSO₄, NaCl-gesättigtem Wasser, gesättigter NaHCO₃-Lösung und nochmals mit NaCl-gesättigtem Wasser gewaschen und mit Na₂SO₄ getrocknet. Das Lösungsmittel wurde im Vakuum entfernt und das Produkt aus Essigester kristallisiert.
Ausbeute: 5,8 g (11,98 mmol) weiße Kristalle, HPLC: 50,78 % B

### 5b) H-Ser(Bzl)-4-Acetyloxamidinobenzylamid x HCl

2 g (4,12 mmol) Boc-Ser(Bzl)-4-Acetyloxamidinobenzylamid wurden mit 30 ml 1 N HCl in Eisessig versetzt. Nach 45 min Stehen bei Raumtemperatur wurde das Lösungsmittel teilweise eingeengt und das Produkt durch Zugabe von Diethylether gefällt, abgesaugt und nochmals mit Diethylether gewaschen. Das Produkt wurde im Vakuum getrocknet.
Ausbeute: 1,6 g (3,8 mmol) weißer Feststoff, HPLC: 28,51 % B

### 5c) Bzls-dSer(tBu)-Ser(Bzl)-4-Acetyloxamidinobenzylamid

0,75 g (2,376 mmol) Bzls-dSer(tBu)-OH und 1 g (2,376 mmol) H-Ser(Bzl)-4-Aceyloxamidinobenzylamid x HCl wurden in 20 ml DMF gelöst und bei 0 °C mit 1,236 g (2,376 mmol) PyBop und 1,033 ml (5,94 mmol) DIEA versetzt. Der Ansatz wurde 30 min bei 0 °C und weitere 4 h bei Raumtemperatur gerührt. Das Lösungsmittel wurde im Vakuum entfernt, der Rückstand in Essigester aufgenommen und jeweils 3 x mit 5 % KESO₄, NaCl-gesättigtem Wasser, gesättigter NaHCO₃-Lösung und nochmals mit NaCl-gesättigtem Wasser gewaschen und anschließend mit Na₂SO₄ getrocknet. Das Lösungsmittel wurde im Vakuum entfernt. Es verblieb ein öliges Rohprodukt, das direkt für den nächsten Syntheseschritt eingesetzt wurde.
Ausbeute: 1,15 g (1,69 mmol) Öl, HPLC: 60,48 %B

### 5d) Bzls-dSer(tBu)-Ser(Bzl)-4-Amidinobenzylamid x Acetat

1 g (1,467 mmol) Bzls-dSer(tBu)-Ser(Bzl)-4-Acetyloxamidinobenzylamid wurden in 50 ml 90 % Essigsäure gelöst, dazu wurden 150 mg Katalysator (10 % Pd/C) gegeben. Der Ansatz wurde 6 h bei Raumtemperatur und Normaldruck mit Wasserstoff hydriert. Anschließend wurde der Katalysator abfiltriert, das Lösungsmittel im Vakuum eingeengt und mit Toluol versetzt. Das Lösungsmittel wurde im Vakuum entfernt, der Vorgang wurde noch 2 x wiederholt. Der verbleibende Rückstand wurde im Vakuum getrocknet und ohne weitere Reinigung für den nächsten Syntheseschritt eingesetzt.
Ausbeute: 0,9 g (1,316 mmol) Öl, HPLC: 49,91 % B.

### 5e) Bzls-dSer-Ser-4-Amidinobenzylamid x TFA

0,2 g Rohprodukt an Bzls-dSer(tBu)-Ser(Bzl)-4-Amidinobenzylamid x Acetat wurden unter Eiskühlung mit 5 ml TFA versetzt. Nach 10 min wurden 500 µl Trifluormethansulfonsäure hinzugefügt. Nach weiteren 5 min wurde das Eisbad entfernt und der Ansatz 20 min bei Raumtemperatur stehen gelassen. Das Produkt wurde durch Zugabe von Diethylether gefällt und abzentrifugiert. Das Präzipitat wurde nochmals mit Diethylether versetzt, aufgeschüttelt und nochmals zentrifugiert. Das Präzipitat wurde mit präparativer reversed-phase HPLC gereinigt.
Ausbeute: 75 mg, HPLC: 24,64 % B
MS: berechnet 477,17 (monoisotopic), gefunden 478,6 [M+H]⁺

### Beispiel 6: 4-(Aminomethyl)Benzylsulfonyl-dSer-Gly-4-Amidinobeazylamid x 2 TFA

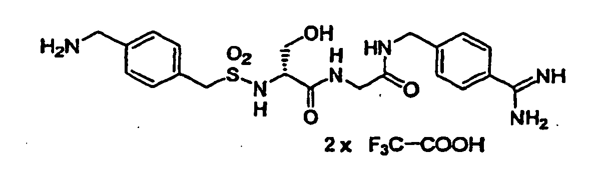

### 6a) 4-Cyano-Benzylsulfonsäure Natrium-Salz

30 g (153 mmol) 4-Cyanobenzylbromid (Aldrich) wurden in 150 ml Wasser suspendiert und nach Zugabe von 21,2 g (168,3 mmol) Na₂SO₃ 8 h unter Rückfluß gekocht. Der Ansatz wurde heiß filtriert und das Wasser im Vakuum etwas eingeengt. Der Ansatz wurde zur Kristallisation über Nacht im Kühlschrank aufbewahrt, danach wurden die Kristalle abgesaugt und nochmals aus Wasser umkristallisiert. Die Kristalle wurden abgesaugt und im Vakuum getrocknet
Ausbeute: 17,1 g (78 mmol), HPLC: 18,24 % B

### 6b) 4-Cyano-Benzylsulfonylchlorid

5 g (22,83 mmol) 4-Cyano-Benzylsulfonsäre Natrium-Salz wurden mit ca. 20 ml Phosphorylchlorid angefeuchtet und mit 5,2 g (25,11 mmol) PCl₅ versetzt und 15 min unter Eiskühlung gerührt. Anschließend wurde der Ansatz 4 h auf 80 °C erwärmt. Danach wurde der Ansatz auf Eis gegossen und 30 min kräftig gerührt, das Produkt schied sich als weißer Feststoff auf dem Eis ab. Nachdem das Eis teilweise aufgetaut war, wurde der Ansatz durch eine Fritte filtriert und das verbleibende Produkt-Eis-Gemisch mehrmals mit Wasser gewaschen. Die verbleibenden Kristalle wurden im Vakuum getrocknet und direkt für den nächsten Syntheseschritt verwendet.
Ausbeute: 3,4 g (15,76 mmol)

### 6c) 4-Cyano-Benzylsulfonyl-dSer(tBu)-OH

1 g (6,2 mmol) H-dSer(tBu)-OH (Bachem) wurden in 50 ml trockenem DCM suspendiert, mit 1,65 ml (13 mmol) Trimethylsilylchlorid und 2,26 ml (13 mmol) DIEA versetzt. Der Ansatz wurde 1 h unter Rückfluß gekocht und im Eisbad abgekühlt. Anschließend wurden 1,47 g (6,82 mmol) 4-Cyano-Benzylsulfonylchlorid und 1,19 ml (6,82 mmol) DIEA innerhalb von 30 min zugesetzt Der Ansatz wurde weitere 15 min unter Eiskühlung und danach für weitere 3 h bei Raumtemperatur gerührt. Das Lösungsmittel wurde im Vakuum entfernt, der Rückstand in Wasser (mit 1 N NaOH auf pH 8,5-9 gebracht) gelöst und 2 x mit Ether extrahiert. Anschließend wurde die wässrige Phase mit 5 % KHSO₄-Lösung angesäuert und 3 x mit Essigester extrahiert. Die vereinte Essigesterphase wurde jeweils 3 x mit 5 % KHSO₄-Lösung und NaCl-gesättigter Lösung gewaschen und mit Na₂SO₄ getrocknet. Das Lösungsmittel wurde im Vakuum entfernt.
Ausbeute: 1,4 g (4,11 mmol Feststoff), HPLC: 48,89 % B

### 6d) 4-Cyano-Benzylsulfonyl-dSer(tBu)-Gly-4-Acetyloxamidinobenzylamid

1 g (2,94 mmol) 4-Cyano-Benzylsulfonyl-dSer(tBu)-OH und 0,884 g (2,94 mmol) H-Gly-4-Acetyloxamidinobenzylamid x HCl (siehe Beispiel 1d) wurden unter Rühren und Eiskühlung in 15 ml DMF gelöst und mit 1,53 g (2,94 mmol) PyBop sowie 1,38 ml (7,94 mmol) DIEA versetzt. Nach 30 min wurde das Eisbad entfernt und der Ansatz weitere 4 h bei Raumtemperatur gerührt. Das DMF wurde im Vakuum eingeengt, der verbleibende Rückstand in Essigester gelöst und jeweils 3 x mit 5 % KHSO₄, NaCl-gesättigtem Wasser, gesättigter NaHCO₃-Lösung und nochmals mit NaCl-gesättigtem Wasser gewaschen und mit Na₂SO₄ getrocknet. Das Lösungsmittel wurde im Vakuum entfernt. Das Rohprodukt wurde ohne weitere Reinigung für den nächsten Syntheseschritt verwendet.
Ausbeute: 1,4 g (2,386 mmol) Öl. HPLC:46,05 % B

### 6e) 4-Cyano-Benzylsulfonyl-dSer(tBu)-Gly-4-Amidinobenzylamid x Acetat

1 g (1,7 mmol) 4-Cyano-Benzylsulfonyl-dSer(tBu)-Gly-4-Acetyloxamidinobenzylamid wurden in 70 ml 90 % Essigsäure gelöst, mit 150 mg Katalysator (10 % Pd auf Aktivkohle) versetzt und 5 h mit Wasserstoff hydriert. Der Katalysator wurde abfiltriert und das Lösungsmittel eingeengt. Der verbleibende Rückstand wurde mit Toluen versetzt, anschließend wurde das Lösungsmittel im Vakuum entfernt. Dieser Vorgang wurde nochmals wiederholt. Der verbleibende Rückstand wurde direkt für den nächsten Reaktionsschritt verwendet
Ausbeute: 0,85 g (1,44 mmol als Acetat-Salz) Feststoff HPLC: 37,55 % B
Ca. 60 mg dieses Rohproduktes wurden mit präparativer HPLC gereinigt.
MS: berechnet 528,2 (monoisotopic), gefunden 530,1 [M+H]⁺

### 6f) 4-Aminomethyl-Benzylsulfonyl-dSer(tBu)-Gly-4-Amidinobenzylamid x 2 TFA

200 mg Rohprodukt an 4-Cyano-Benzylsulfonyl-dSer(tBu)-Gly-4-Amidinobenzylamid x Acetat wurden in 50 ml 90 % Essigsäure und 5 ml 1 N HCl gelöst, mit 40 mg Katalysator (10 % Pd auf Aktivkohle) versetzt und über Nacht bei 40 °C mit Wasserstoff hydriert Der Katalysator wurde abfiltriert und das Lösungsmittel im Vakuum eingeengt. Der verbleibende Rückstand wurde mit präparativer reversed phase HPLC gereinigt.
Ausbeute: 75 mg (als 2 x TFA-Salz) Feststoff HPLC: 26,05 % B
MS: berechnet 532,25 (monoisotopic), gefunden 533,7 [M+H]⁺

### 6g) 4-Aminomethyl-Benzylsulfonyl-dSer-Gly-4-Amidinobenzylamid x 2 TFA

25 mg (0,033 mmol) 4-Aminomethyl-Benzylsulfonyl-dSer(tBu)-Gly-4-Amidinobenzylamid x 2 TFA wurden mit 0,2 ml Wasser und 1,8 ml TFA versetzt. Der Ansatz wurde 60 min bei Raumtemperatur belassen und das Lösungsmittel im Vakuum eingeengt. Der Rückstand wurde mit ca. 10 ml Wasser versetzt und lyophilisiert.
Ausbeute: 20 mg (schwach gelblicher Feststoff) HPLC: 15,4 % B
MS: berechnet 476,18 (monoisotopic), gefunden 477,5 [M+H]⁺

Tabelle 2: Hemmkonstanten (Kᵢ in µM) und Halbwertszeiten (t_{½} in h) der Elimination (β-Phase) in Ratten nach intravenöser Gabe von 1 mg/kg für Inhibitoren der allgemeinen Struktur. Die Bestimmung der Hemmkonstanten (Kᵢ und t_{½}) erfolgte für uPA wie in Stürzebecher et al., (1997) J Med Chem Vol. 40, 3091-3099 beschrieben und für Plasmin, Trypsin und Thrombin analog hierzu.

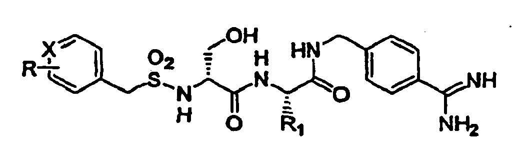

| R | X | R1 | Kᵢ (µM) | | | | t_{½} (h) |
|---|---|---|---|---|---|---|---|
| | | | uPA | Plasmin | Trypsin | Thrombin | |
| H | CH | H | 0,036 | 11 | 0,15 | 13 | 0,29 |
| 3-COOMe | CH | H | 0,12 | 28 | 0,29 | 42 | n.b.* |
| 3-COOH | CH | H | 0,16 | 59 | 0,72 | 150 | 1,3 |
| 4-COOMe | CH | H | 0,62 | 17 | 0,18 | 9,4 | n.b. |
| 4-COOH | CH | H | 0,15 | 35 | 0,48 | 170 | 2,0 |
| 2-COOMe | CH | H | 0,083 | 38 | 0,40 | 4,0 | n.b. |
| 2-COOH | CH | H | 0,37 | 220 | 2,4 | 56 | n.b. |
| 4-COOH | CH | CH₃ | 0,038 | 3,0 | 0,013 | 2,3 | 0,66 |
| 3-COOH | CH | CH₃ | 0,030 | 4,7 | 0,021 | 8,3 | 0,42 |
| 4-CN | CH | CH | 0,089 | 27 | 0,34 | 8,5 | n.b. |
| 4-(NH₂-CH₂) | CH | H | 0,12 | 7,4 | 0,28 | 8,0 | n.b. |
| H | CH | CH₂-OH | 0,025 | 0,75 | 0,022 | 14 | 0,50 |
| H | CH | CH₂-O(Bzl) | 0,028 | 0,27 | 0,0068 | 0,48 | n.b. |
| H | CH | CH₂-NH₂ | 0,036 | 0,81 | 0,021 | 0,78 | 0,40 |
| H | CH | CH(OH)CH₃ | 0,11 | 1,4 | 0,03 | 4,0 | n.b. |
| H | CH | CH(OBzl)CH₃ | 0,061 | 1,1 | 0,011 | 0,10 | n.b. |
| 3-COOH | CH | CH₂-OH | 0,075 | 4,2 | 0,058 | 200 | 0,43 |
| 4-COOH | CH | CH₂-OH | 0,23 | 6,2 | 0,10 | 120 | 0,43 |
| 4-OOOMe | CH | CH₂-OH | 0,23 | 0,96 | 0,020 | 4,2 | n.b. |
| 4-Cl | CH | H | 0,032 | 32 | 0,35 | 7,9 | n.b. |
| 4-Me | CH | H | 0,058 | 18 | 0,21 | 8,0 | n.b. |
| 4-F | CH | H | 0,031 | 20 | 0,11 | 7,9 | n.b. |
| 3,4-Di-Cl | CH | H | 0,11 | 32 | 0,60 | 8,3 | n.b. |
| H | N | H | 0,10 | 37 | 0,41 | 1,6 | n.b. |

| | | | | | | | |
|---|---|---|---|---|---|---|---|
| *n.b. = nicht bestimmt | | | | | | | |

### Beispiel 7: Benzylsulfonyl-dSer-Lys-4-Amidinobenzylamid x 2 TFA

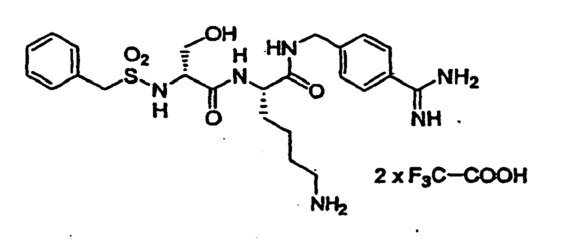

### 7a) Boc-Lys(Tfa)-4-Acetyloxamidinobenzylamid

5 g (14,61 mmol) Boc-Lys(Tfa)-OH wurden in 100 ml THF gelöst und bei -15 °C mit 1,767 ml (16,10 mmol) NMM und 1,899 ml (14,61 mmol) CKIBE versetzt. Der Ansatz wurde 10 min bei -15°C gerührt, dann wurden 3,74 g (15,33 mmol) 4-(Acetyloxamidino)Benzylamin x HCl (hergestellt wie in WO 01/96286 A2 beschrieben) und nochmals 1,767 ml (16,10 mmol) NMM hinzugefügt. Der Ansatz wurde eine weitere Stunde bei -15 °C und über Nacht bei Raumtemperatur gerührt. Das Lösungsmittel wurde im Vakuum entfernt, der Ansatz in Essigester aufgenommen und jeweils 3 x mit 5 % KHSO₄, NaCl-gesättigtem Wasser, gesättigter NaHCO₃-Lösung und nochmals mit NaCl-gesättigtem Wasser gewaschen und mit Na₂SO₄ getrocknet. Das Lösungsmittel wurde im Vakuum entfernt und das Produkt aus Essigester kristallisiert.
Ausbeute: 6,82 g (12,83 mmol) weiße Kristalle, HPLC: 43,87 % B

### 7b) H-Lys(Tfa)-4-Acetyloxamidinobenzylamid x HCl

5 g (9,41 mmol) Boc-Lys(Tfa)4-Acetyloxamidinobenzylamid wurden in wenig Eisessig angelöst und anschliessend mit 100 ml 1 N HCl in Eisessig versetzt. Nach 45 min Stehen bei Raumtemperatur wurde das Lösungsmittel teilweise eingeengt und das Produkt durch Zugabe von Diethylether gefällt, abgesaugt und nochmals mit Diethylether gewaschen. Das Produkt wurde im Vakuum getrocknet
Ausbeute: 4,65 g (10,78 mmol) weißer Feststoff, HPLC: 25,52 % B

### 7c) Bzls-dSer(tBu)-Lys(Tfa)-4-Acetyloxamidinobenzylamid

1,93 g (6,107 mmol) Bzls-dSer(tBu)-OH und 3 g (6,412 mmol) H-Lys(Tfa)-4-Acetyloxamidinobenzylamid x HCl wurden in 30 ml Acetonitril gelöst und bei 0 °C mit 3,337 g (6,412 mmol) PyBop und 3,187 ml (18,32 mmol) DIEA versetzt. Der Ansatz wurde 30 min bei 0 °C und weitere 4 h bei Raumtemperatur gerührt. Das Lösungsmittel wurde im Vakuum entfernt, der Rückstand in Essigester aufgenommen und jeweils 3 x mit 5% KHSO₄, NaCl-gesättigtem Wasser, gesättigter NaHCO₃-Lösung und nochmals mit NaCl-gesättigtem Wasser gewaschen und anschließend mit Na₂SO₄ getrocknet. Das Lösungsmittel wurde im Vakuum entfernt. Es verblieb ein leicht gelbes, amorphes Rohprodukt, das ohne weitere Reinigung direkt für den nächsten Syntheseschritt eingesetzt wurde.
Ausbeute: 5,88 g (Rohprodukt), HPLC: 52,93 % B

### 7d) Bzls-dSer(tBu)-Lys(Tfa)-4-Amidinobenzylamid x Acetat

5,88 g (Rohprodukt) Bzls-dSer(tBu)-Lys(Tfa)-4-(Acetyloxamidino) Benzylamid wurden in 150 ml 90 % Essigsäure gelöst, dazu wurden 500 mg Katalysator (10 % Pd/C) gegeben. Der Ansatz wurde 6 h bei Raumtemperatur und Normaldruck mit Wasserstoff hydriert. Anschließend wurde der Katalysator abfiltriert, das Lösungsmittel teilweise eingeengt und das Produkt durch Zugabe von Diethylether gefällt, abgesaugt und nochmals mit Diethylether gewaschen. Der weisse, kristalline Niederschlag wurde im Vakuum getrocknet.
Ausbeute: 4,36 g (5,962 mmol), HPLC: 43,50 % B.

### 7e) Bzls-dSer-Lys-4-Amidinobenzylamid x 2 TFA

0,2 g Rohprodukt an Bzls-dSer(tBu)-Lys(Tfa)-4-Amidinobenzylamid x Acetat wurden unter Eiskühlung mit 5 ml 1M wässriger Piperidinlösung versetzt und 3 h gerührt. Anschliessend wurden 45 ml TFA zugegeben. Nach 1 h Rühren bei Raumtemperatur wurde das Lösungsmittel im Vakuum eingeengt, mit Toluol versetzt und das Lösungsmittel erneut im Vakuum entfernt. Dieser Vorgang wurde noch 2 x wiederholt. Der verbleibende Rückstand wurde im Vakuum getrocknet und ohne weitere Reinigung mit präparativer reversed-phase HPLC gereinigt.
Ausbeute: 65 mg, HPLC: 21,19 % B
MS: berechnet 574,26 (monoisotopic), gefunden 574,3 [M+H]⁺

### Beispiel 8: Benzylsulfonyl-dSer-Arg-4-Amidinobenzylamid x 2 TFA

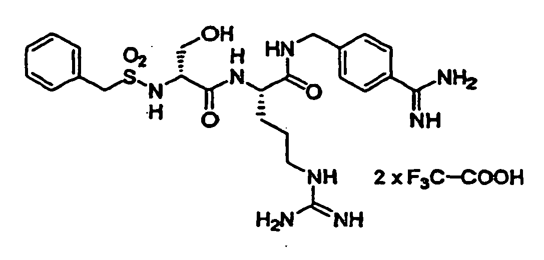

### 8a) Boc-Arg(Boc)₂-4-Acetyloxamidinobenzylamid

0,5 g (1,05 mmol) Boc-Arg(Boc)₂-OH wurden in 25 ml THF gelöst und bei -15 °C mit 122 µl (1,11 mmol) NMM und 137 µl (1,05 mmol) CKIBE versetzt. Der Ansatz wurde 10 min bei -15 °C gerührt, dann wurden 0,274 g (1,11 mmol) 4-(Acetyloxamidino)Benzylamin x HCl (hergestellt wie in WO 01/96286 A2 beschrieben) und nochmals 122 µl (1,11 mmol) NMM hinzugefügt Der Ansatz wurde eine weitere Stunde bei -15 °C und über Nacht bei Raumtemperatur gerührt. Das Lösungsmittel wurde im Vakuum entfernt, der Ansatz in Essigester aufgenommen und jeweils 3 x mit 5 % KHSO₄, NaCl-gesättigtem Wasser, gesättigter NaHCO₃-Lösung und nochmals mit NaCl-gesättigtem Wasser gewaschen und mit Na₂SO₄ getrocknet Das Lösungsmittel wurde im Vakuum entfernt, wobei das Produkt als weisse, amorphe Substanz anfiel.
Ausbeute: 0,654 g (0,985 mmol), HPLC: 48,89 % B

### 8b) H-Arg-4-Acetyloxamidinobenzylamid x HCl

0,65 g (0,979 mmol) Boc-Arg(Boc)₂-4-Acetyloxamidinobenzylamid wurden in wenig Eisessig angelöst und anschliessend mit 100 ml 1 N HCl in Eisessig versetzt. Nach 45 min Stehen bei Raumtemperatur wurde das Lösungsmittel teilweise eingeengt und das Produkt durch Zugabe von Diethylether gefällt, abgesaugt und nochmals mit Diethylether gewaschen. Das Produkt wurde im Vakuum getrocknet.
Ausbeute: 0,459 g (0,971 mmol) weißer Feststoff, HPLC: 17,01 % B

### 8c) Bzls-dSer(tBu)-Arg-4-(Acetyloxamidino)Benzylamid

0,2 g (0,634 mmol) Bzls-dSer(tBu)-OH und 0,3 g (0,634 mmol) H-Arg-4-Acetyloxamidinobenzylamid x HCl wurden in 30 ml DMF gelöst und bei 0 °C mit 0,33 g (0,634 mmol) PyBop und 331 µl (1,902 mmol) DIEA versetzt. Der Ansatz wurde 30 min bei 0 °C und weitere 4 h bei Raumtemperatur gerührt. Das Lösungsmittel wurde im Vakuum entfernt, der Rückstand in Essigester aufgenommen und jeweils 2 x mit 5 % KHSO₄, mit NaCl-gesättigtem Wasser gewaschen und anschließend mit Na₂SO₄ getrocknet. Das Lösungsmittel wurde im Vakuum entfernt. Es verblieb ein leicht gelbes Öl, das direkt für den nächsten Syntheseschritt eingesetzt wurde.
Ausbeute: 0,724 g (Öl), HPLC: 38,88 % B

### 8d) Bzls-dSer(tBu)-Arg-4-Amidinobenzylamid x 2 Acetat

0,724 g (Rohprodukt) Bzls-dSer(tBu)-Arg-4-Acetyloxamidinobenzylamid wurden in 30 ml 90 % Essigsäure gelöst, dazu wurden 100 mg Katalysator (10 % Pd/C) gegeben. Der Ansatz wurde 6 h bei Raumtemperatur und Normaldruck mit Wasserstoff hydriert. Anschließend wurde der Katalysator abfiltriert, das Lösungsmittel teilweise eingeengt und das Produkt durch Zugabe von Diethylether gefällt, abgesaugt und nochmals mit Diethylether gewaschen. Der weisse, kristalline Niederschlag wurde im Vakuum getrocknet.
Ausbeute: 0,367 g (0,508 mmol), HPLC: 31,66 % B.

### 8e) Bzls-dSer-Arg-4-Amidinobenzylamid x 2 TFA

140 mg (0,194 mmol) Benzylsulfonyl-dSer(tBu)-Gly-4-Amidinobenzylamid x 2 AcOH wurden mit 5 ml 90 % TFA versetzt. Der Ansatz wurde 60 min bei Raumtemperatur belassen, anschliessend das Lösungsmittel teilweise eingeengt und das Produkt durch Zugabe von Diethylether gefällt, abgesaugt und nochmals mit Diethylether gewaschen. Der weisse, kristalline Niederschlag wurde im Vakuum getrocknet und mit präparativer reversed-phase HPLC gereinigt.
Ausbeute: 74 mg (0,055 mmol) HPLC: 22,15 % B
MS: berechnet 546,65 (monoisotopic), gefunden 547,34 [M+H]⁺

Tabelle 3: Hemmkonstanten (Kᵢ in µM) und Halbwertszeiten (t_{½} in h) der Elimination (β-Phase) in Ratten nach intravenöser Gabe von 1 mg/kg für Inhibitoren der allgemeinen Struktur. Die Bestimmung der Hemmkonstanten (Kᵢ und t_{½}) erfolgte für uPA wie in Stürzebecher et al., (1997) Vol. 40, 3091-3099 beschrieben und für Plasmin, Trypsin und Thrombin analog hierzu.

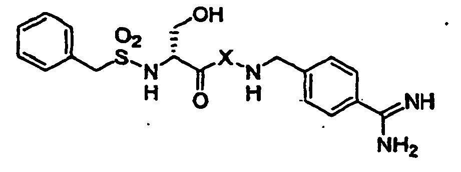

| X | Kᵢ (µM) | | | | t_{½} (h) |
|---|---|---|---|---|---|
| | uPA | Plasmin | Trypsin | Thrombin | |
| Lys | 0,024 | 0,36 | 0,0068 | 4,3 | 0,7 |
| Arg | 0,0089 | 0,2 | 0,007 | 4,7 | 0,6 |

### Beispiel: 9 Benzylsulfanyl-dSer-Lys(CO-O-PEG5000-OMe)-4-Amidinobenzylamid x Acetat

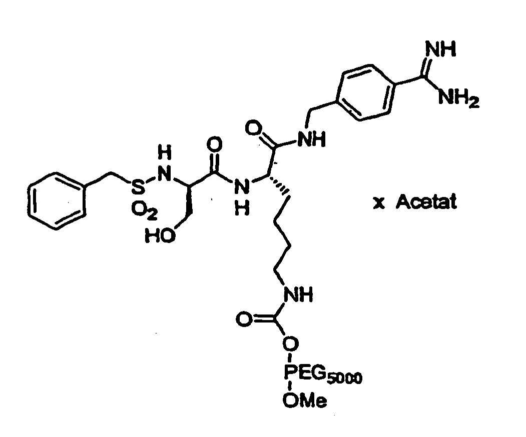

224 mg (0,3 mmol) Benzylsulfonyl-dSer-Lys-4-Amidinobenzylamid x 2 TFA wurden in 20 ml DMF gelöst und bei Raumtemperatur mit 1 g (0,2 mmol) Methoxypolyethylenglykol-p-Niritrophenylcarbonate (Molekulargewicht 5000 Da, Sigma) und 52 µl (0,3 mmol) DIEA versetzt. Nach 1 h wurden nochmals 20 µl DIEA hinzugegeben. Nach 4 h wurde das DMF im Vakuum entfernt, der Rückstand in wenig Methanol gelöst und mit einem großen Volumen an Isopropanol versetzt und im Eis aufbewahrt. Das ausgefallene Produkt wurde abgesaugt und auf der Fritte mit reichlich Isopropanol und anschließend noch mit Diethylether gewaschen. Das Rohprodukt (ca. 1 g) wurde im Vakuum getrocknet und mit einem Ionenaustauscher gereinigt Dazu wurde das Rohprodukt in Wasser gelöst und auf eine Säule (5 cm x 20 cm, Fractogel EMD COO-, equilibriert mit Wasser) aufgetragen. Zuerst wurde die Säule mit 1000 ml Wasser gewaschen und danach das Produkt mit 2 mM Ammoniumacetat-Lösung eluiert. Die Produktenthaltenden Fraktionen (HPLC-Kontrolle, Elution bei 44,96 % B) wurden vereint und das Wasser teilweise eingeengt. Das Produkt wurde insgesamt 3 x aus Wasser lyophilisiert.
Ausbeute: 590 mg, HPLC: 44,96 % B

| Kᵢ (µM) | | | | t_{½} (h) |
|---|---|---|---|---|
| uPA | Plasmin | Trypsin | Thrombin | |
| 0,095 | 0,73 | 0,034 | 1,7 | 1,2 |

### Beispiel 10: Bzls-dSer-Lys(CO-CH₂-O-CH₂-CO-NH-CH₂-CH₂-Hexaethylen-glycol-CH₂-CH₂-NH₂)-4-Amidinobenzylamid x 2 TFA

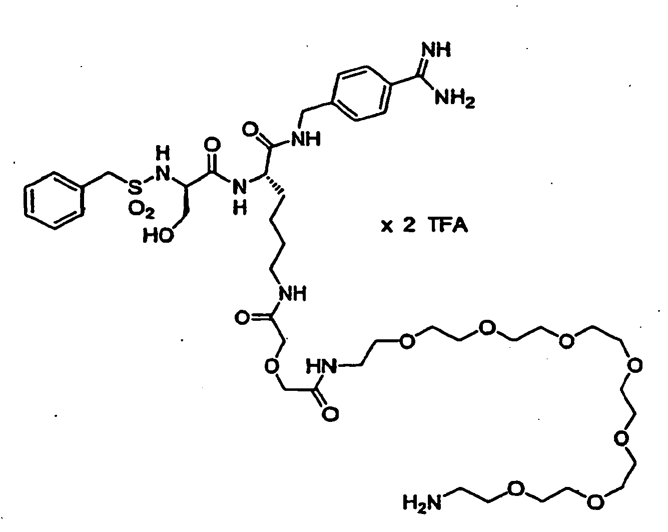

0,392 g (ca. 0,478 mmol) Rohprodukt an Bzls-dSer-Lys-4-Amidinobenzylamid x 2 TFMSA und 280 mg (0,478 mmol) O-(N-Boc-2-Aminoethyl)-O'-(N-Diglycolyl)-2-Aminoethyl)-Hexaethylenglycol (Novabiochem) wurden in 15 ml DMF gelöst Unter Eiskühlung worden 0,249 g (0,478 mmol) PyBop und 250 µl (1,434 mmol) DIEA hinzugegeben. Der Ansatz wurde 15 min unter Eiskühlung und weitere 4 h bei Raumtemperatur gerührt. Danach wurde das Lösungsmittel im Vakuum eingeengt und der Rückstand mit 2 ml Wasser und 18 ml TFA versetzt. Der Ansatz wurde 1 h bei Raumtemperatur gerührt und danach das Lösungsmittel im Vakuum entfernt. Der Rückstand wurde mit Toluen versetzt und das Lösungsmittel wieder im Vakuum entfernt Dieser Vorgang wurde nochmals wiederholt Der Rückstand wurde in wenig Methanol angelöst und das Produkt durch Zugabe von Diethylether gefällt, abgesaugt und mit präparativer HPLC gereinigt.
Ausbeute: 245 mg, HPLC: 26,87 % B
MS: berechnet 984,48 (monoisotopic), gefunden 985,6 [M+H]⁺

| Kᵢ (µM) | | | | t_{½} (h) |
|---|---|---|---|---|
| uPA | Plasmin | Trypsin | Thrombin | |
| 0,042 | 0,53 | 0,0047 | 1,4 | 0,88 |

### Beispiel 11: Benzylsulfonyl-dDap-Gly-4-Amba

Die Verbindung wird unter Anwendung der dem Fachmann bekannten Standardmethoden synthetisiert. Die Hemmkonstanten sind wie folgt

| Kᵢ (µM) | | | | t_{½} (h) |
|---|---|---|---|---|
| uPA | Plasmin | Trypsin | Thrombin | |
| 0,18 | 9,6 | 0,18 | 10 | n.b. |

### Beispiel 12: Benzylsulfonyl-dSer-His-4-Amba

Die Verbindung wird unter Anwendung der dem Fachmann bekannten Standardmethoden synthetisiert. Die Hemmkonstanten sind wie folgt:

| Kᵢ (µM) | | | | t_{½} (h) |
|---|---|---|---|---|
| uPA | Plasmin | Trypsin | Thrombin | |
| 0,11 | 0,40 | 0,025 | 8,5 | n.b. |

### Beispiel 13: 4(HOOC-CH₂₎-Benzylsnlfonyl-dSer-Gly-4-Amba

Die Verbindung wird unter Anwendung der dem Fachmann bekannten Standardmethoden synthetisiert Die Hemmkonstanten sind wie folgt:

| Kᵢ (µM) | | | | t_{½} (h) |
|---|---|---|---|---|
| uPA | Plasmin | Trypsin | Thrombin | |
| 0,13 | 27 | 0,3 | 60 | n.b. |

### Beispiel 14: Hemmung der Metastasierung im Tiermodell

Der Einfluß des Inhibitors Benzylsulfonyl-dSer-Ser-4-Amidinobenzylanmid auf die Metastasierung wurde an weiblichen Mäusen (Stamm CD1 nu/nu, ca 25 g Körpergewicht, Charles River, Sulzfeld) untersucht. Den Mäusen wurden 106 Zellen einer lacZ-markierten menschlichen Fibrosarkom-Zelllinie (HT1080 AN PKZ12 K15-1, gelöst in 200 µl PBS) i.v. appliziert (Krüger et al., Cancer Metastasis Rev. 1998-99, 17, 285-294 und Krüger et al, Oncogene 1998, 16, 2419-2423). Die Mäuse der behandelten Gruppe (n = 17) erhielten ab dem Tag -1 (ein Tag vor der Tumorzell-Inokulation) bis zum 21. Tag danach (insgesamt 23 Tage) täglich 2 i.p.-Gaben (je 1,5 mg/kg) des Inhibitors. Die Mäuse der Kontrollgruppe (n = 10) erhielten entsprechend 200 µl Pyrogen-freies Wasser mit 5 % (v/v) Ethanol. Am Tag 22 wurden die Mäuse getötet, die Lungen wurden in 2 % Formalin und 0,2 % Glutaraldehyd fixiert, danach wurden die Lungen mit X-Gel (5-Br-4-Cl-3-Indolyl-β-D-Galaktosid) angefärbt und die Anzahl der Lungenmetastasen bestimmt.

Ergebnis: Die Anzahl der Lungenmetastasen in der mit dem Inhibitor Benzylsulfonyl-dSer-Ser-4-Amidinobenzylamid behandelten Gruppe wurde im Vergleich zur Kontrollgruppe (100 %) auf 4,6 % reduziert.

### Verwendete Abkürzungen:

- Ac: Acetyl
- Boc: tert-Butyloxycarbonyl
- Bzl: Benzyl
- Bzls: Benzylsulfonyl
- CKIBE: Chlorkohlensäureisobutylester
- Dab: α,γ-Diammobuttersäure
- Dap: α,β-Diaminopropionsäure
- DIEA: Diisopropylethylamin
- DMF: N,N-Dimethylformamid
- dSer: D-serin
- iBu: iso-butyl
- i.V.: im Vakuum
- n.b.: nicht bestimmt
- NMM: N-Methylmorpholin
- PyBOP: Benzotriazol-1-yl-N-oxy-tris(pyrrolidino)phosphoniumhexafluorophosphat
- TEA: Triethylamin
- TFA: Trifluoressigsäure
- Tfa: Trifluoracetyl
- TFMSA: Trifluormethansulfonsäure
- THF: Tetrahydrofuran

## Patentansprüche

1. Verbindung der allgemeinen Formel **I** 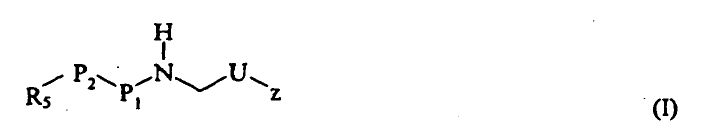 wobei 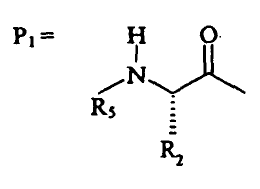 und 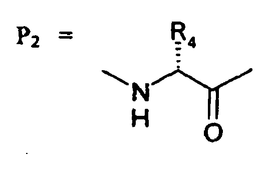 ist;
R₂ ein H, ein verzweigter oder unverzweigter Alkylrest mit 1 bis 8 C-Atomen vorzugsweise mit 1 bis 3 C-Atomen, oder -(CH₂)_{c}COOR₈ mit c = 1, 2, 3 oder 4, wobei R₈ H oder ein verzweigter oder unverzweigter Alkylrest mit vorzugsweise 1 bis 6 C-Atomen, insbesondere 1 bis 3 C-Atomen, vor allem Ethyl, ist oder -(CH₂)_{d}-OR₉ mit d = 1, 2, 3 oder 4, wobei R₉ H ist, oder -(CH₂)ₑR₁₀, -(CH₂)ₑOR₁₀, -(CH₂)ₑSR₁₀, -(CH₂)ₑ-Guanidino, -(CH₂)ₑ-Imidazol oder -(CH₂)ₑNHR₁₀ mit e = 1, 2, 3, 4 oder 5 ist, wobei R₁₀ H, ein verzweigter oder unverzweigter Alkylrest mit 1-16, insbesondere 1-8, vor allem 1-3 C-Atomen oder ein substituierter oder unsubstituierter Aryl-, Heteroaryl-, Aralkyl- oder Heteroaralkylrest ist, wobei der Alkylrest vorzugsweise 1 bis 16, insbesondere 1 bis 8, vor allem 1 bis 3 C-Atome, und der Aryl- oder Heteroarylrest vorzugsweise 4 bis 14, insbesondere 6 bis 10, vor allem 6 C-Atome und vorzugsweise 1 bis 3 N als Heteroatom besitzt, oder -(CH₂)ₖO-CO-OR₁₆ mit k = 1,2, 3, 4, 5, 6, 7 oder 8 ist, wobei R₁₆ ein verzweigtes oder unverzweigtes Alkyl mit 1-16, vorzugsweise 1-8, insbesondere 1-4, vor allem 1-2 C-Atomen, ein substituierter oder unsubstituierter Aryl-, Heteroaryl-, Aralkyl- oder Heteroaralkylrest, ein Adamantyl-, ein Campher-, ein Cyclohexylmethylrest, vorzugsweise Benzyl, ist; und
R₅ -SO₂R₁₂ ist, wobei R₁₂ ein substituierter Aralkylrest, vorzugsweise Benzyl, ist;
wobei R₅ mit einer geladenen oder ungeladenen Gruppe, vorzugsweise einer -(CH₂)ⱼCOOR₁₃, -(CH₂)ⱼSO₂R₁₃, -(CH₂)ⱼNH₂, -(CH₂)ⱼ-Amidino-, -(CH₂)ⱼ-Hydroxyamidino- oder -(CH₂)ⱼ-Guanidino-Gruppe mit j = 0, 1 oder 2 modifiziert ist, wobei R₁₃ H oder ein Alkylrest mit vorzugsweise 1 bis 6 C-Atomen, insbesondere Ethyl, ist, oder
R₂ -(CH₂)_{c}COOR₈ mit c = 1, 2, 3 oder 4, wobei R₈ H oder ein verzweigter oder unverzweigter Alkylrest mit vorzugsweise 1 bis 6 C-Atomen, insbesondere 1 bis 3 C-Atomen, vor allem Ethyl, ist oder -(CH₂)ₑSR₁₀, -(CH₂)ₑ-Guanidino, -(CH₂)ₑ-Imidazol oder -(CH₂)ₑNHR₁₀ mit e = 1, 2, 3, 4 oder 5 ist, wobei R₁₀ H, ein verzweigter oder unverzweigter Alkylrest mit 1-16, insbesondere 1-8, vor allem 1-3 C-Atomen oder ein substituierter oder unsubstituierter Aryl-, Heteroaryl-, Aralkyl- oder Heteroaralkylrest ist, wobei der Alkylrest vorzugsweise 1 bis 16, insbesondere 1 bis 8, vor allem 1 bis 3 C-Atome, und der Aryl- oder Heteroarylrest vorzugsweise 4 bis 14, insbesondere 6 bis 10, vor allem 6 C-Atome und vorzugsweise 1 bis 3 N als Heteroatom besitzt, oder -(CH₂)ₖO-CO-OR₁₆ mit k = 1, 2, 3, 4, 5, 6, 7 oder 8 ist, wobei R₁₆ ein verzweigtes oder unverzweigtes Alkyl mit 1-16, vorzugsweise 1-8, insbesondere 1-4, vor allem 1-2 C-Atomen, ein substituierter oder unsubstituierter Aryl-, Heteroaryl-, Aralkyl- oder Heteroaralkylrest, ein Adamantyl-, -ein Campher-, ein Cyclohexylmethylrest, vorzugsweise Benzyl, ist, und
R₅ -SO₂R₁₂ ist, wobei R₁₂ ein substituierter oder unsubstituierter Aralkylrest, vorzugsweise Benzyl, ist;
wobei R₅ mit einer geladenen oder ungeladenen Gruppe, vorzugsweise einer -(CH₂)ⱼCOOR₁₃, -(CH₂)ⱼSO₂R₁₃, -(CH₂)ⱼNH₂, -(CH₂)ⱼ-Amidino-, -(CH₂)ⱼHydroxyamidino- oder -(CH₂)ⱼ-Guanidino-Gruppe mit j = 0; 1 oder 2 modifiziert sein kann, wobei R₁₃ H oder ein Alkylrest mit vorzugsweise 1 bis 6 C-Atomen, insbesondere Ethyl, ist;
und wobei P₁ in der Formel I in der L-Konfiguration vorliegt;
R₄ -(CH₂)_{f}OR₁₁ mit f = 1 ist, wobei R₁₁ H ist und wobei P₂ in der Formel I in der D-Konfiguration vorliegt;
U ein Phenylrest ist;
Z in 3- oder 4-Position vorkommt und eine Aminomethyl-, eine Guanidino- oder eine Amidinogruppe 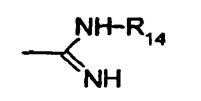
ist, wobei R₁₄ H, OH, NH₂, -COR₁₅ oder -COOR₁₅ ist, wobei R₁₅ ein verzweigter oder unverzweigter Alkylrest mit 1 bis 16, vorzugsweise 1 bis 8, insbesondere 1 bis 4, vor allem 1 bis 2 C-Atomen oder ein substituierter oder unsubstituierter Aryl- oder Heteroaryl-, Aralkyl- oder Heteroaralkylrest ist, wobei der Alkylrest vorzugsweise 1 bis 16, insbesondere I bis 8, vor allem 1 bis 4 und besonders bevorzugt 1 bis 2 C-Atome und der Aryl- oder Heteroarylrest vorzugsweise 4 bis 14, insbesondere 6 bis 10, vor allem 6 C-Atome und vorzugsweise 1 bis 3 N als Heteroatom besitzt;
**dadurch gekennzeichnet, dass** ein oder mehrere geladene Reste vorzugsweise abgeleitet von -COOH, -CH(COOH)₂, -SO₂H, NH₂, einer Amidino-, Hydroxyamidino-, Amidrazono- oder Guanidinogruppe in den Resten R₂ oder R₅ vorhanden sind;
oder eine Verbindung der allgemeinen Formel I in Form eines Prodrugs oder in Form ihres Salzes.

2. Verbindung nach Anspruch 1, wobei zusätzlich eine mit einer Amino- oder Carboxylgruppe funktionalisierte Oligo- oder Polyalkylenglycolkette, insbesondere eine Poly- oder Oligoethylenglycol- oder Poly- oder Oligopropylenglycolkette direkt an eine funktionelle Gruppe von R₂ insbesondere über eine -NH- oder eine -CO-Gruppe unter Ausbildung einer Amidbindung an R₂ gekoppelt ist, wobei die Oligo- oder Polyalkylenglycolkette zumindest an beiden Enden eine funktionelle Gruppe, insbesondere eine substituierte oder unsubstituierte Amino- und/oder Carboxylgruppe aufweist, oder wobei die Oligo- oder Polyalkylenglycolkette zumindest an einem Ende eine funktionelle Gruppe, insbesondere eine substituierte oder unsubstituiert Amino- und/oder Carboxylgruppe aufweist und am anderen Ende als Alkylether mit 1-4 C-Atomen, insbesondere als Methylether vorliegt,
wobei R₂ vorzugsweise
(a) -(CH₂)ₙ-NH₂ mit n gleich 1-5, vorzugsweise 4 oder
(b) -(CH₂)ₙ-COOH mit n gleich 1-5, vorzugsweise 1-3 ist.

3. Verbindung nach Anspruch 1 oder 2, wobei P₁ nach Kopplung des Oligo- oder Polyalkylenglykols die allgemeine Formel II aufweist 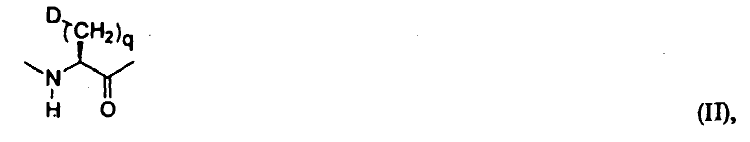 wobei q gleich 0, 1, 2, 3, 4 oder 5 ist und D gleich Formel III ist
E-F-G- (III),
wobei
wenn E gleich eine H₂N-, HOOC-(CH₂)ₙ-CO-NH-, HOOC- oder H₂N-(CH₂)ₙ-NH-CO-Gruppe ist, F gleich ein Oligo- oder Polyalkylenglycol der allgemeinen Formel -(CH₂)_{d}-[O-CH₂-CH₂]ᵥO-(CH₂)ₘ-(NH-CO-CH₂-O-CH₂)ₖ- oder -(CH₂)_{d}-[O-CH(CH₃)-CH₂]ᵥ-O-(CH₂)ₘ-(NH-CO-CH₂-O-CH₂)ₖ- mit d = 1, 2, 3 oder 4, v = eine ganze Zahl von 1 bis 1000, bevorzugt 1 bis 250, m = 0, 1, 2, 3 oder 4 und k = 0 oder 1 ist oder
wenn E gleich eine CH₃-O-Gruppe ist, F gleich eine Oligo- oder Polyalkylenglycolkette der allgemeinen Formel -(CH₂)_{d}-[O-CH₂-CH₂]ᵥO-(CH₂)ₘ-(NH-CO-CH₂-O-CH₂)ₖ₋ oder -(CH₂)_{d}-[O-CH(CH₃)-CH₂]ᵥ-O-(CH₂)ₘ-(NH-CO-CH₂-O-CH₂)ₖ- mit d = 1, 2, 3 oder 4, v = eine ganze Zahl von 1 bis 1000, bevorzugt 1 bis 250, m = 0, 1, 2, 3 oder 4 und k = 0 oder 1 ist;
und G gleich -CO-NH- oder -NH-CO- ist.

4. Verbindung nach mindestens einem der Ansprüche 1 bis 3, wobei U an 1, 2 oder 3 Positionen vorzugsweise mit einem Halogen, insbesondere Fluor oder Chlor, oder einem Methyl-, Ethyl-, Propyl-, Methoxy-, Ethoxy-, oder Propoxyrest substituiert ist.

5. Verbindung nach mindestens einem der Ansprüche 1 bis 4, wobei eine Carboxylgruppe als Ester, bevorzugt als Ethylester, geschützt vorliegt.

6. Verbindung nach mindestens einem der Ansprüche 1 bis 5 in Form eines Prodrugs, wobei R₉ und/oder R₁₁ in diesem Fall ein Alkylcarbonyl-, Aralkylcarbonyl-, Alkyloxycarbonyl- oder Aralkyloxycarbonyl-Rest ist, wobei der Alkylrest vorzugsweise 1 bis 6, insbesondere 1 bis 4 C-Atome und der Arylrest vorzugsweise 5 bis 8, insbesondere 6 C-Atome besitzt.

7. Verbindung nach mindestens einem der Ansprüche 1 bis 6, **dadurch gekennzeichnet, dass** im Amidinobenzylamidrest die Amidinogruppe in 4-Position steht und dass P₂ von der Aminosäure D-Ser abstammt und P₁ von Glycin, Alanin, Serin, Asparaginsäure oder Glutaminsäure abstammt und dass R₅ ein unsubstituierter oder mit einer Carboxylgruppe oder Carboxyalkylgruppe versehener Aryl- oder Aralkylsulfonyl-Rest mit 1 bis 16, vorzugsweise 1 bis 8, insbesondere 1 bis 4, vor allem 1 bis 2 C-Atomen im Alkylrest und 6 bis 14, vorzugsweise 6 bis 10, insbesondere 6 C-Atomen im Arylrest ist.

8. Verbindung nach mindestens einem der Ansprüche 1 bis 6, **dadurch gekennzeichnet, dass** im Amidinobenzylamidrest die Amidinogruppe in 4-Position steht und dass P₂ die Aminosäure D-Ser und P₁ eine natürliche oder künstliche, unsubstituierte oder substituierte basische Aminosäure in der L-Konfiguration bedeutet, beispielsweise Lys, homoLys, Arg, norArg, homoArg, His, Orn, Orn(2-Imidazolinyl), Dab, 4-[(2-Amino)Pyrimidinyl]Buttersäure, Dap, Ala[3-(2-Pyrrolidinyl)], Ala[3-Pyrrolidinyl-(2-N-Amidino)], Ala[3-(N-Piperazine-4-N-amidino], Ala(4-Pip), Ala[4-Pip(N-amidino)], homoAla(4-Pip), Ala[3-Pip(N-amidino)], homoAla(3-Pip), homoAla[4-Pip(N-amidino)], Ala-(3-guanidino), Phe(3-Amidino), Phe(4-Amidino), Phe(3-NH₂), Phe(4-NH₂), Phe(3-Guanidino), Phe(4-Guanidino), Phe[4-(2-imidazolinyl)], Phe[3-CH₂-(guanidino)], Phe[4-CH₂-(guanidino)], homoPhe(3-Amidino), homoPhe(4-Amidino), hPhe(3-NH₂), hPhe(4-NH₂), hPhe(3-Guanidino), hPhe(4-Guanidino), cis-Cha(4-NH₂), trans-Cha(4-NH₂), cis-homoCha(4-NH₂), trans-homoCha(4-NH₂), trans-Cha(4-CH₂NH₂), trans-homocha(4-CH₂NH₂), und **dadurch gekennzeichnet, dass** R₅ ein mit einer Sulfonylgruppe versehener Aryl- oder Aralkylsulfonyl-Rest mit 1 bis 16, vorzugsweise 1 bis 8, insbesondere 1 bis 4, vor allem 1 bis 2 C-Atomen im Alkylrest und 6 bis 14, vorzugsweise 6 bis 10, insbesondere 6 C-Atomen im Arylrest ist, der an die Aminogruppe des D-Ser gebunden ist.

9. Verbindung nach Anspruch 8, **dadurch gekennzeichnet, dass** P₁ die Aminosäure Lys oder Arg bedeutet.

10. Verbindung nach mindestens einem der Ansprüche 1 bis 9, **dadurch gekennzeichnet, dass** der Substituent am substituierten Aryl-, Heteroaryl-, Aralkyl- oder Heteroaralkylrest ein Halogen, vorzugsweise Fluor, Chlor oder Brom, insbesondere Fluor oder Chlor, ist.

11. Verbindung nach mindestens einem der Ansprüche 1 bis 10, **dadurch gekennzeichnet, dass** eine Verbindung der allgemeinen Formel I die folgende Struktur aufweist: 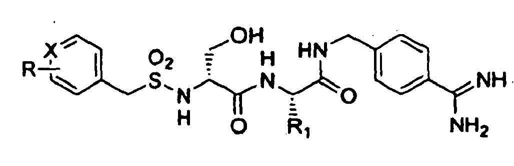 mit R gleich COOH, HOOC-(CH₂)ₚ-, R₁₈OOC-(CH₂)ₚ- mit p = 1 und 2 und R₁₈ = Methyl oder Ethyl, oder COOMe in ortho-, meta- oder para- oder H und X gleich CH und R₁ gleich H; oder
R gleich 4-COOH oder 3-COOH mit X gleich CH und R₁ gleich H, CH₃ oder CH₂-OH; oder
R gleich 4-COOH oder 3-COOH mit X gleich CH und R₁ gleich H, CH₃ oder CH₂-OH; oder
R gleich 4-CN mit X gleich CH und R₁ gleich CH₃; oder
R gleich 4-(NH₂-CH₂) mit X gleich CH und R₁ gleich H; oder
R gleich 4-COOMe mit X gleich CH und R₁ gleich CH₂-OH.

12. Verbindung nach mindestens einem der Ansprüche 1 bis 10, **dadurch gekennzeichnet, dass** eine Verbindung der allgemeinen Formel I eine der folgenden Strukturen aufweist: 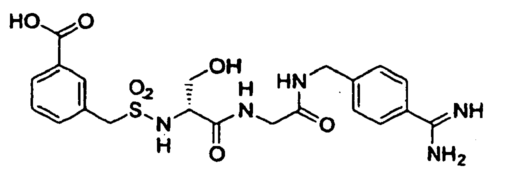 oder 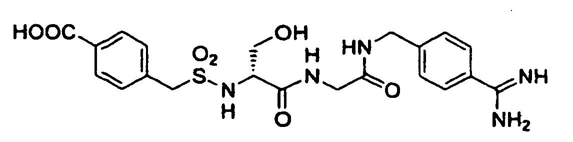 oder

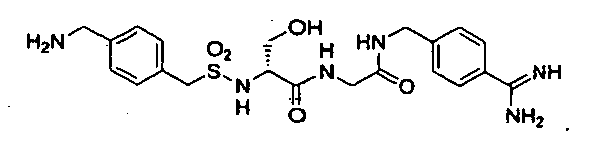

13. Verbindung nach mindestens einem der Ansprüche 1 bis 10, **dadurch gekennzeichnet, dass** eine Verbindung der allgemeinen Formel I eine der folgenden Strukturen aufweist: 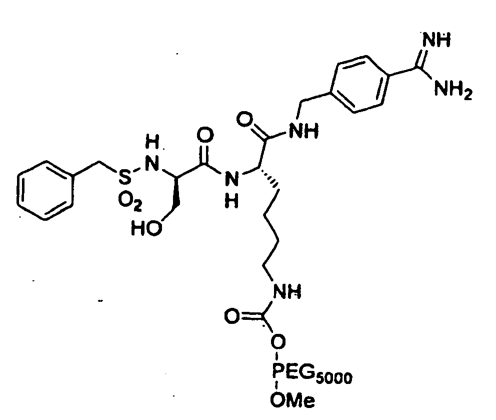 oder 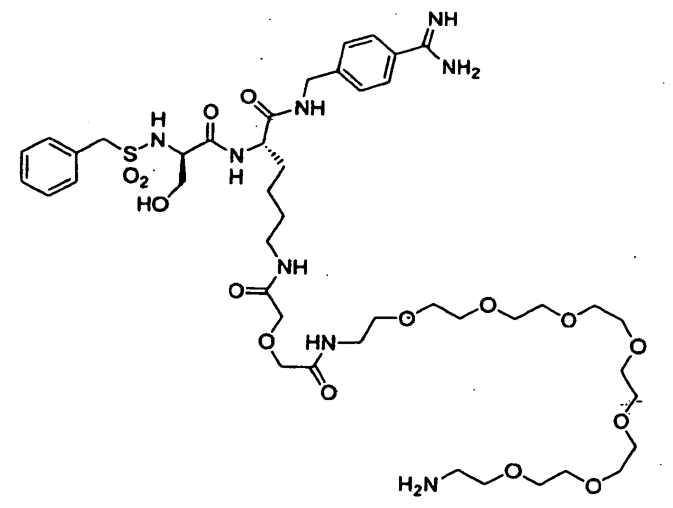 oder 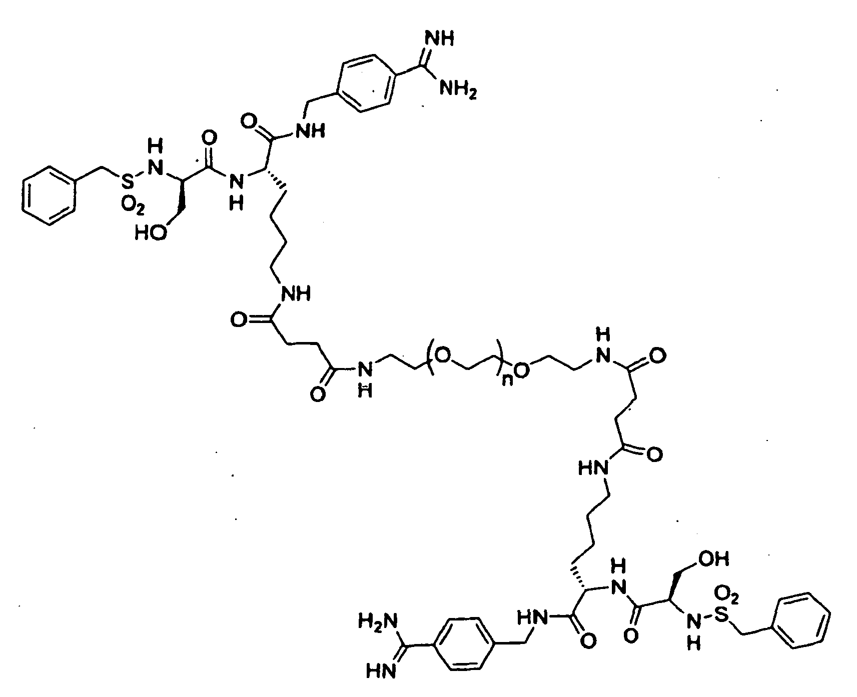 mit n = 2 bis 250.

14. Verbindung nach mindestens einem der Ansprüche 1-13, **dadurch gekennzeichnet, dass** die Verbindungen bevorzugt als Salze vorliegen, vorzugsweise mit Mineralsäuren, bevorzugt als Hydrochloride, oder vorzugsweise als Salze mit geeigneten organischen Säuren.

15. Verbindung nach Anspruch 14, **dadurch gekennzeichnet, dass** bevorzugte Salze von Mineralsäuren auch Sulfate sind und geeignete organische Säuren beispielsweise Essigsäure, Ameisensäure, Methylsulfonsäure, Bernsteinsäure, Apfelsäure oder Trifluoressigsäure sind, wobei bevorzugte Salze von organischen Säuren Acetate sind.

16. Verfahren zur Herstellung einer Verbindung gemäß mindestens einem der Ansprüche 1 bis 15, **dadurch gekennzeichnet, dass** sequentiell die entsprechenden Aminosäuren an ein 4-Acetyloxamidinobeozylalamin angekoppelt werden, wobei die N-terminale Aminosäure entweder den R₅-Rest bereits trägt oder dieser anschließend daran gebunden wird.

17. Arzneimittel enthaltend eine Verbindung gemäß mindestens einem der Ansprüche 1 bis 15 sowie pharmazeutisch geeignete Hilfs- und/oder Zusatzstoffe.

18. Arzneimittel nach Anspruch 17, wobei das Arzneimittel in Form einer Tablette, eines Dragees, einer Kapsel, eines Pellets, Suppositoriums, einer Lösung, insbesondere einer Injektions- oder Infusionslösung, von Augen-, Nasen und Ohrentropfen, eines Safts, einer Kapsel, einer Emulsion oder Suspension, eines Globuli, Styli, Aerosols, Puders, einer Paste, Creme oder Salbe eingesetzt wird.

19. Verwendung einer Verbindung gemäß mindestens einem der Ansprüche 1 bis 15 zur Herstellung eines Medikaments zur Therapie oder Prophylaxe eines Tumors, insbesondere zur Reduzierung der Bildung von Tumormetastasen, bevorzugt in oraler, subkutaner, intravenöser oder transdermaler Form.

20. Verwendung einer Verbindung gemäß mindestens einem der Ansprüche 1 bis 15 zur Herstellung eines Mittels zur Diagnose eines Tumors.

## Claims

1. A compound of the general formula I 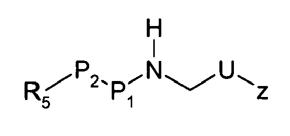 wherein 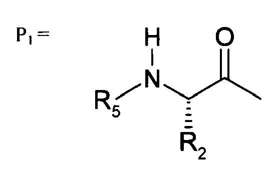 and 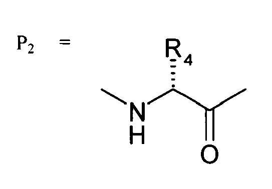
R₂ is an H, a branched or unbranched alkyl radical having from 1 to 8 C atoms, preferably having from 1 to 3 C atoms, or
-(CH₂)_{c}COOR₈, in which c = 1, 2, 3 or 4, where R₈ is H or a branched or unbranched alkyl radical preferably having from 1 to 6 C atoms, in particular from 1 to 3 C atoms, especially ethyl, or
-(CH₂)_{d}-OR₉, in which d = 1, 2, 3 or 4, where R₉ is H, or
-(CH₂)ₑR₁₀, -(CH₂)ₑ-OR₁₀, -(CH₂)ₑ-SR₁₀, -(CH₂)ₑ-guanidino, -(CH₂)ₑ-imidazole or -(CH₂)ₑNHR₁₀, in which e = 1, 2, 3, 4 or 5, where R₁₀ is H, a branched or unbranched alkyl radical having 1-16, in particular 1-8, especially 1-3, C atoms, or a substituted or unsubstituted aryl, heteroaryl, aralkyl or heteroaralkyl radical, where the alkyl radical preferably possesses from 1 to 16, in particular from 1 to 8, especially from 1 to 3, C atoms, and the aryl or heteroaryl radical preferably possesses from 4 to 14, in particular from 6 to 10, especially 6, C atoms, and preferably from 1 to 3 N as heteroatom, or
-(CH₂)ₖO-CO-OR₁₆, in which k = 1, 2, 3, 4, 5, 6, 7 or 8, where R₁₆ is a branched or unbranched alkyl having 1-16, preferably 1-8, in particular 1-4, especially 1-2, C atoms, a substituted or unsubstituted aryl, heteroaryl, aralkyl or heteroaralkyl radical, or an adamantyl, a camphor or a cyclohexylmethyl radical, preferably benzyl;and
R₅ is -SO₂R₁₂, where R₁₂ is a aralkyl radical, preferably benzyl,
where R₅ is modified with a charged or uncharged group, preferably with a -(CH₂)ⱼCOOR₁₃, -(CH₂)ⱼSO₂R₁₃, -(CH₂)ⱼNH₂, -(CH₂)ⱼ-amidino, -(CH₂)ⱼ-hydroxyamidino or -(CH₂)ⱼ-guanidino group in which j = 0, 1 or 2, where R₁₃ is H or an alkyl radical preferably having from 1 to 6 C atoms, in particular ethyl; or
R₂ -(CH₂)_{c}COOR₈, in which c = 1, 2, 3 or 4, where R₈ is H or a branched or unbranched alkyl radical preferably having from 1 to 6 C atoms, in particular from 1 to 3 C atoms, especially ethyl, or
-(CH₂)ₑ-SR₁₀, -(CH₂)ₑ-guanidino, -(CH₂)ₑ-imidazole or -(CH₂)ₑNHR₁₀, in which e = 1, 2, 3, 4 or 5, where R₁₀ is H, a branched or unbranched alkyl radical having 1-16, in particular 1-8, especially 1-3, C atoms, or a substituted or unsubstituted aryl, heteroaryl, aralkyl or heteroaralkyl radical, where the alkyl radical preferably possesses from 1 to 16, in particular from 1 to 8, especially from 1 to 3, C atoms, and the aryl or heteroaryl radical preferably possesses from 4 to 14, in particular from 6 to 10, especially 6, C atoms, and preferably from 1 to 3 N as heteroatom, or -(CH₂)ₖO-CO-OR₁₆, in which k = 1, 2, 3, 4, 5, 6, 7 or 8, where R₁₆ is a branched or unbranched alkyl having 1-16, preferably 1-8, in particular 1-4, especially 1-2, C atoms, a substituted or unsubstituted aryl, heteroaryl, aralkyl or heteroaralkyl radical, or an adamantyl, a camphor or a cyclohexylmethyl radical, preferably benzyl; and
R₅ is -SO₂R₁₂, where R₁₂ is a branched or unbranched aralkyl radical, preferably benzyl, where R₅ can be modified with a charged or uncharged group, preferably a -(CH₂)ⱼCOOR₁₃, -(CH₂)ⱼSO₂R_{13,} -(CH₂)ⱼNH₂, -(CH₂)ⱼ-amidino, -(CH₂)ⱼ-hydroxyamidino or -(CH₂)ⱼ-guanidino group in which j = 0, 1 or 2, where R₁₃ is H or an alkyl radical preferably having from 1 to 6 C atoms, in particular ethyl;
and wherein P1 in formula I is present in the L-configuration
R₄ is -(CH₂)_{f}OR₁₁, in which f = 1, where R₁₁ is H, and wherein P₂ being present in the D configuration in the formula I;
U is a phenyl radical;
Z occurs in the 3 or 4 position and is an aminomethyl, a guanidino or an amidino group 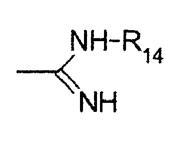
where R₁₄ is H, OH, NH₂, -COR₁₅ or -COOR₁₅, where R₁₅ is a branched or unbranched alkyl radical having from 1 to 16, preferably from 1 to 8, in particular from 1 to 4, especially from 1 to 2, C atoms or a substituted or unsubstituted aryl or heteroaryl, aralkyl or heteroaralkyl radical, where the alkyl radical preferably possesses from 1 to 16, in particular from 1 to 8, especially from 1 to 4, and particularly preferably from 1 to 2, C atoms and the aryl or heteroaryl radical preferably possesses from 4 to 14, in particular from 6 to 10, especially 6, C atoms and, preferably, from 1 to 3 N as heteroatom;
**characterized in that** one or more charged radicals, preferably derived from -COOH, -CH(COOH)₂, -SO₂H, NH₂, an amidino, hydroxyamidino, amidrazono or guanidino group, is/are present in the radicals R₂ or R₅;
or a compound of the general formula I in the form of a prodrug or in the form of its salt.

2. A compound as claimed in claim 1, in which an amino group-functionalized or carboxyl group-functionalized oligo- or polyalkylene glycol chain, in particular a poly- or oligoethylene glycol chain or poly- or oligopropylene glycol chain, is coupled directly to a functional group of R₂, in particular by way of an -NH or a -CO group, with the formation of an amide bond at R₂, with the oligo- or polyalkylene glycol chain possessing a functional group, in particular a substituted or unsubstituted amino group and/or carboxyl group, at least at both ends, or with the oligo- or polyalkylene glycol chain possessing a functional group, in particular a substituted or unsubstituted amino group and/or carboxyl group, at one end and being present, at the other end, as an alkyl ether having 1-4 C atoms, in particular as methyl ether,
with R₂ preferably being
(a) -(CH₂)ₙ-NH₂ in which n is 1-5, preferably 4, or
(b) -(CH₂)ₙ-COOH in which n is 1-5, preferably 1-3.

3. A compound as claimed in claim 1 or 2, wherein, after coupling the oligo- or polyalkylene glycol, P₁ has the general formula II 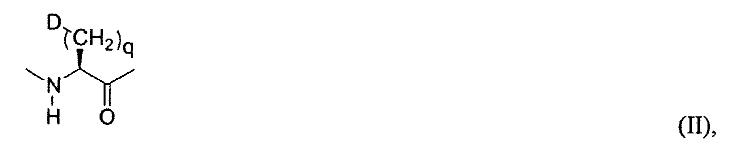 where q is 0, 1, 2, 3, 4 or 5 and D is formula III
E - F - G - (III)
where, when E is an H₂N, HOOC-(CH₂)ₙ-CO-NH, HOOC or H₂N-(CH₂)ₙ-NH-CO group, F is an oligo- or polyalkylene glycol of the general formula -(CH₂)_{d}-[O-CH₂-CH₂]ᵥO-(CH₂)ₘ-(NH-CO-CH₂-O-CH₂)ₖ- or -(CH₂)_{d}-[O-CH(CH₃)-CH₂]ᵥO-(CH₂)ₘ-(NH-CO-CH₂-O-CH₂)ₖ-, in which d = 1, 2, 3 or 4, v = an integer from 1 to 1000, preferably from 2 to 250, m = 0, 1, 2, 3 or 4, and k = 0 or 1, or, when E is a CH₃-O group, F is an oligo- or polyalkylene glycol chain of the general formula -(CH₂)_{d}-[O-CH₂-CH₂]ᵥO-(CH₂)ₘ-(NH-CO-CH₂-O-CH₂)ₖ- or -(CH₂)_{d}-[O-CH(CH₃)-CH₂]ᵥ-O-(CH₂)ₘ-(NH-CO-CH₂-O-CH₂)ₖ-, in which d = 1, 2, 3 or 4, v = an integer from 1 to 1000, preferably from 1 to 250, m = 0, 1, 2, 3 or 4, and k = 0 or 1;
and G is -CO-NH- or -NH-CO-.

4. A compound as claimed in at least one of claims 1 to 3, wherein U is substituted at 1, 2 or 3 positions, preferably by a halogen, in particular fluorine or chlorine, or a methyl, ethyl, propyl, methoxy, ethoxy or propoxy radical.

5. A compound as claimed in at least one of claims 1 to 4, wherein a carboxyl group is protected as an ester, preferably as an ethyl ester.

6. A compound as claimed in at least one of claims 1 to 5 in the form of a prodrug, with R₉ and/or R₁₁ in this case being an alkylcarbonyl, aralkylcarbonyl, alkyloxycarbonyl or aralkoxycarbonyl radical, with the alkyl radical preferably having from 1 to 6, in particular from 1 to 4, C atoms and the aryl radical preferably having from 5 to 8, in particular 6, C atoms.

7. A compound as claimed in at least one of claims 1 to 6, **characterized in that** the amidino group is in the 4 position in the amidinobenzylamide radical and **in that** P₂ is derived from the amino acid D-Ser and P₁ is derived from glycine, alanine, serine, aspartic acid or glutamic acid, and **in that** R₅ is an unsubstituted aryl- or aralkylsulfonyl radical, or such a radical provided with a carboxyl group or carboxyalkyl group, having from 1 to 16, preferably from 1 to 8, in particular from 1 to 4, especially from 1 to 2, C atoms in the alkyl radical and from 6 to 14, preferably from 6 to 10, in particular 6, C atoms in the aryl radical.

8. A compound as claimed in at least one of claims 1 to 6, **characterized in that** the amidino group is in the 4 position in the amidinobenzylamide radical and **in that** P₂ is the amino acid D-Ser and P₁ is a natural or artificial, unsubstituted or substituted, basic amino acid in the L configuration, for example Lys, homoLys, Arg, norArg, homoArg, His, Orn, Orn(2-imidazolinyl), Dab, 4-[(2-amino)pyrimidinyl]butyric acid, Dap, Ala[3-(2-pyrrolidinyl)], Ala[3-pyrrolidinyl-(2-N-amidino)], Ala[3-(N-piperazine-4-N-amidino], Ala(4-Pip), Ala[4-Pip(N-amidino)], homoAla(4-Pip), Ala[3-Pip(N-amidino)], homoAla(3-Pip), homoAla[4-Pip(N-amidino)], Ala-(3-guanidino), Phe(3-amidino), Phe(4-amidino), Phe(3-NH₂), Phe(4-NH₂), Phe(3-guanidino), Phe(4-guanidino), Phe[4-(2-imidazolinyl)], Phe[3-CH₂-(guanidino)], Phe[4-CH₂-(guanidino)], homoPhe(3-amidino), homoPhe(4-amidino), hPhe(3-NH₂), hPhe(4-NH₂), hPhe(3-guanidino), hPhe(4-guanidino), cis-Cha(4-NH₂), trans-Cha(4-NH₂), cis-homoCha(4-NH₂), trans-homoCha (4-NH₂), trans-Cha (4-CH₂NH₂) and trans-homoCha (4-CH₂NH₂), and **in that** R₅ is a sulfonyl group-provided aryl- or aralkylsulfonyl radical having from 1 to 16, preferably from 1 to 8, in particular from 1 to 4, especially from 1 to 2, C atoms in the alkyl radical and from 6 to 14, preferably from 6 to 10, in particular 6, C atoms in the aryl radical, which is bonded to the amino group of the D-Ser.

9. A compound as claimed in claim 8, **characterized in that** P₁ is the amino acid Lys or Arg.

10. A compound as claimed in at least one of claims 1 to 9, **characterized in that** the substituent on the substituted aryl, heteroaryl, aralkyl or heteroaralkyl radical is a halogen, preferably fluorine, chlorine or bromine, in particular fluorine or chlorine.

11. A compound as claimed in at least one of claims 1 to 10, **characterized in that** a compound of the general formula I has the following structure: 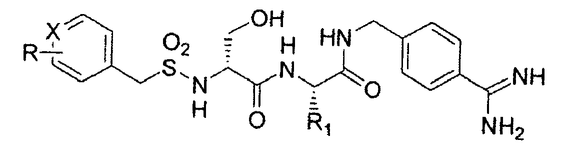 in which R is COOH, HOOC-(CH₂)ₚ- or R₁₈OOC-(CH₂)ₚ-in which p = 1 and 2 and R₁₈ = methyl or ethyl, or COOMe in ortho, meta or para, or H, and X is CH and R₁ is H; or
R is 4-COOH or 3-COOH, with X being CH and R₁ being H, CH₃ or CH₂-OH; or
R is 4-COOH or 3-COOH, with X being CH and R₁ being H, CH₃ or CH₂-OH; or
R is 4-CN, with X being CH and R₁ being CH₃; or
R is 4-(NH₂-CH₂), with X being CH and R₁ being H; or
R is 4-COOMe, with X being CH and R₁ being CH₂-OH.

12. A compound as claimed in at least one of claims 1 to 10, **characterized in that** a compound of the general formula I has one of the following structures: 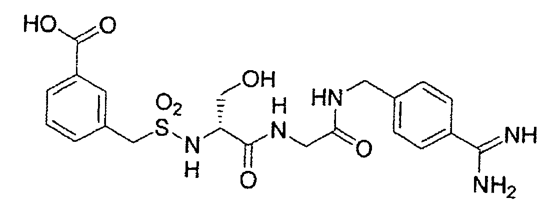 or 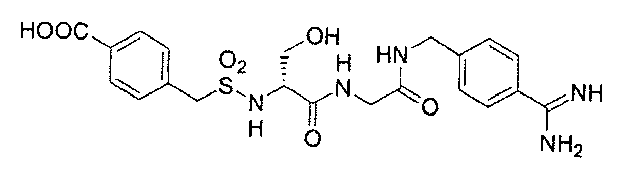 or

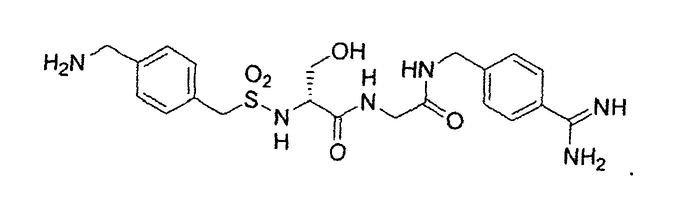

13. A compound as claimed in at least one of claims 1 to 10, **characterized in that** a compound of the general formula I has one of the following structures: 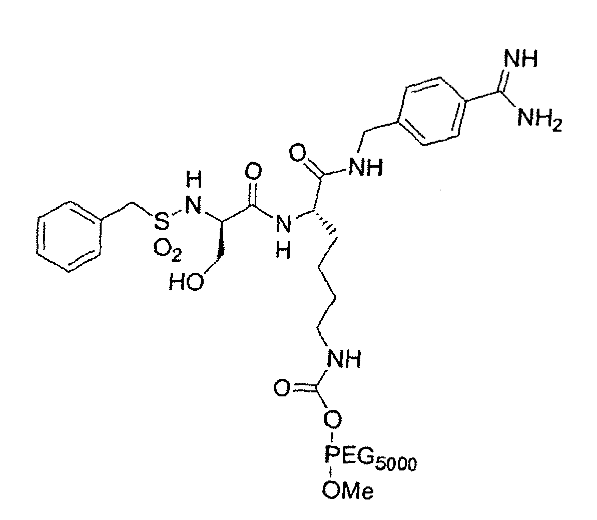 or 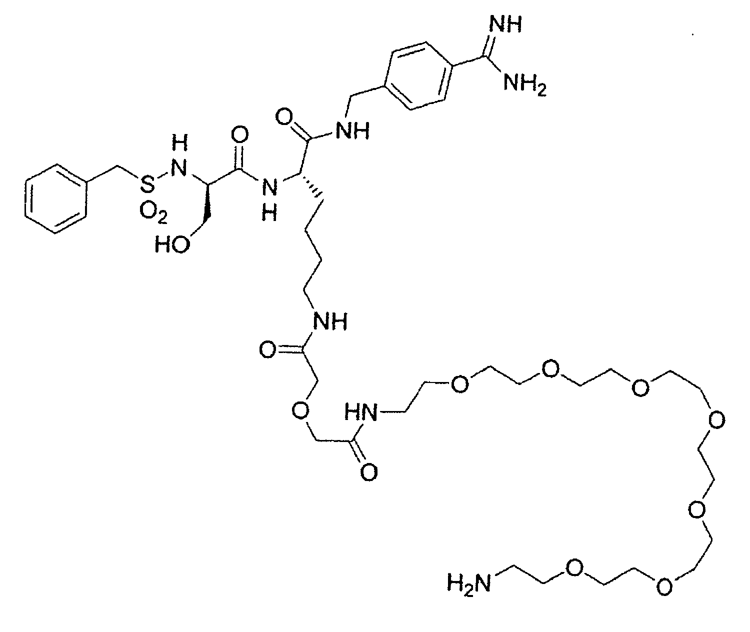 or 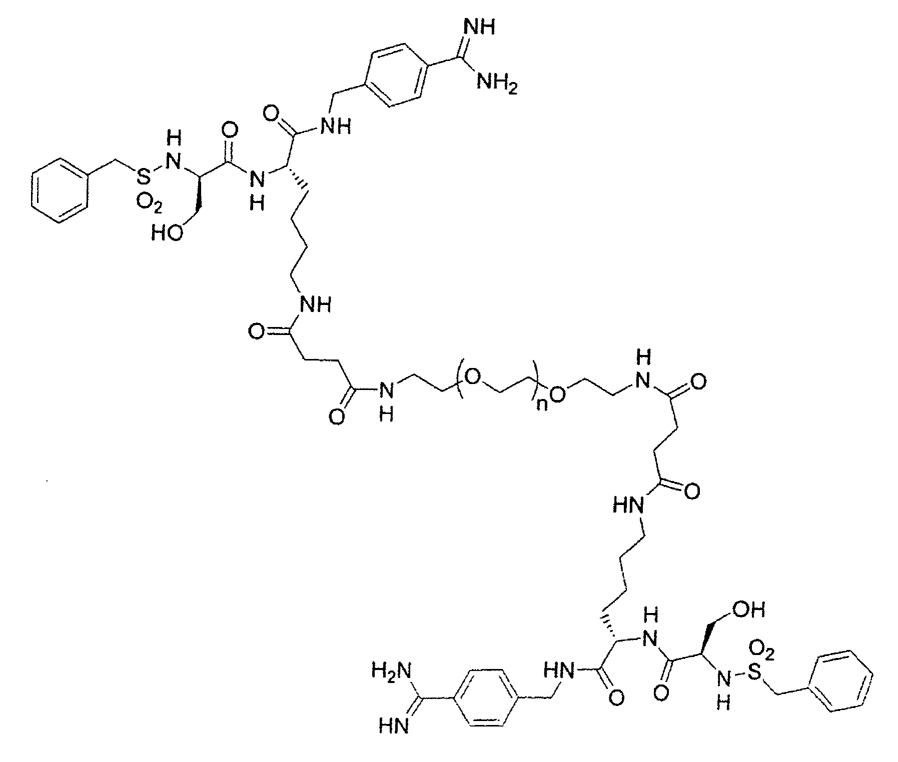 in which n = 2 to 250.

14. A compound as claimed in at least one of claims 1-13, **characterized in that** the compounds are preferably present as salts, preferably with mineral acids, preferably as hydrochloride, or preferably as salts with suitable organic acids.

15. A compound as claimed in claim 14, **characterized in that** preferred salts of mineral acids are also sulfates and suitable organic acids are, for example, acetic acid, formic acid, methylsulfonic acid, succinic acid, malic acid or trifluoroacetic acid, with preferred salts of organic acids being acetates.

16. A process for preparing a compound as claimed in at least one of claims 1 to 15, **characterized in that** the appropriate amino acids are sequentially coupled to a 4-acetyloxamidinobenzylamine, with either the N-terminal amino acid already carrying the R₅ radical or with this radical subsequently being bonded to it.

17. A pharmaceutical which comprises a compound as claimed in at least one of claims 1 to 15 and also pharmaceutically suitable auxiliary substances and/or additives.

18. A pharmaceutical as claimed in claim 17, wherein the pharmaceutical is used in the form of a tablet, of a sugar-coated tablet, of a capsule, of a pellet, of a suppository, of a solution, in particular of an injection solution or infusion solution, of eyedrops, nose drops and ear drops, of a juice, of a capsule, of an emulsion or suspension, of a globule, of a stylus, of an aerosol, of a powder, of a paste, of a cream or of an ointment.

19. The use of a compound as claimed in at least one of claims 1 to 15, for the preparation of a medicament for the therapy or prophylaxis of a tumor, in particular for reducing the formation of tumor metastases, preferably in oral, subcutaneous, intravenous or transdermal form.

20. The use of a compound as claimed in at least one of claims 1 to 15 for the preparation of a pharm,aveutical for the diagnosis a tumor.

## Revendications

1. Composé de formule générale 1 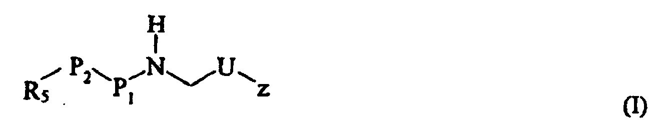 dans laquelle 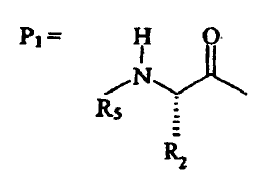 et 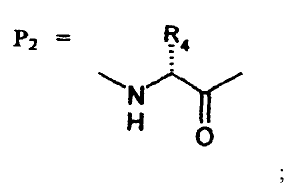 R₂ représente
un atome d'hydrogène,
un radical alkyle ramifié ou non ramifié comportant de 1 à 8 atomes de carbone, de préférence 1 à 3 atomes de carbone ou
-(CH₂)cCOOR₈ avec c = 1, 2, 3 ou 4, R₈ représentant un atome d'hydrogène ou un radical alkyle ramifié ou non ramifié comportant de préférence de 1 à 6 atomes de carbone, en particulier de 1 à 3 atomes de carbone, en particulier le radical éthyle ou
-(CH₂)_{d}-OR₉ avec d = 1, 2, 3 ou 4, R₉ étant un atome d'hydrogène ou
-(CH2)ₑ-R₁₀, -(CH₂)e-OR₁₀, -(CH₂)ₑ-SR₁₀, -(CH₂)ₑ-guanidino, -(CH₂)ₑ-imidazole ou -(CH₂)ₑNHR₁₀ avec e = 1, 2, 3, 4 ou 5, R₁₀ représentant un atome d'hydrogène, un radical alkyle ramifié ou non ramifié comportant de 1 à 16, en particulier de 1 à 8, en particulier de 1 à 3 atomes de carbone ou un radical aryle, hétéroaryle, aralkyle ou hétéroaralkyle substitué ou non substitué, le radical alkyle comportant de préférence de 1 à 16, en particulier de 1 à 8, en particulier de 1 à 3 atomes de carbone et le radical aryle ou hétéroaryle, de préférence de 4 à 14, en particulier de 6 à 10, en particulier 6 atomes de carbone et de préférence 1 à 3N en tant qu'hétéroatome ou
-(CH₂)ₖO-CO-OR₁₆ avec k = 1, 2, 3, 4, 5, 6, 7 ou 8, R16 représentant un groupe alkyle ramifié ou non ramifié avec 1 à 16, de préférence 1 à 8, en particulier 1 à 4, en particulier 1 à 2 atomes de carbone, un radical aryle, hétéroaryle, aralkyle ou hétéroaralkyle substitué ou non substitué, un radical adamantyle, un radical camphre ou un radical cyclohexylméthyle, de préférence benzyle; et
R₅ représente
-SO₂R₁₂, R₁₂ représentant un radical aralkyle substitué, de préférence benzyle;
où R₅ est modifié par un groupe chargé ou non chargé, de préférence un groupe -(CH₂)ⱼCOOR₁₃, -(CH₂)ⱼSO2R₁₃, -(CH₂)ⱼNH₂, -(CH₂)ⱼ-amidino, -(CH₂)ⱼ-hydroxyamidino ou -(CH₂)ⱼ-guanidino avec j = 0, 1 ou 2, R₁₃ représentant un atome d'hydrogène ou un radical alkyle comportant, de préférence, de 1 à 6 atomes de carbone, en particulier un radical éthyle
ou
R₂ représente
-(CH₂)_{c}COOR₈ avec c = 1, 2, 3 ou 4, R₈ représentant un atome d'hydrogène ou un radical alkyle ramifié ou non ramifié comportant, de préférence de 1 à 6 atomes de carbone, en particulier de 1 à 3 atomes de carbone, en particulier le radical éthyle ou un groupe -(CH₂)ₑsR₁₀, -(CH₂)ₑ-guanidino, -(CH₂)ₑ-imidazole ou -(CH₂)ₑNHR10 avec e = 1, 2, 3, 4 ou 5, R₁₀ représentant un atome d'hydrogène, un radical alkyle ramifié ou non ramifié comportant de 1 à 16, en particulier de 1 à 8, en particulier de 1 à 3 atomes de carbone ou un radical aryle, hétéroaryle, aralkyle ou hétéroaralkyle substitué ou non substitué, le radical alkyle comportant de préférence de 1 à 16, en particulier de 1 à 8, en particulier de 1 à 3 atomes de carbone et le radical aryle ou hétéroaryle, de préférence de 4 à 14, en particulier de 6 à 10, en particulier 6 atomes de carbone et de préférence 1 à 3 N en tant qu'hétéroatome ou
-(CH₂)ₖO-CO-OR₁₆ avec k = 1, 2, 3, 4, 5, 6, 7 ou 8, R₁₆ représentant un groupe alkyle ramifié ou non ramifié avec 1 à 16, de préférence 1 à 8, en particulier 1 à 4, en particulier 1 à 2 atomes de carbone, un radical aryle, hétéroaryle, aralkyle ou hétéroaralkyle substitué ou non substitué, un radical adamantyle, un radical camphre ou un radical cyclohexylméthyle, de préférence benzyle; et
R₅ représente
-SO₂R₁₂, R₁₂ représentant un radical aralkyle substitué ou non substitué, de préférence benzyle; R₅ étant modifié par un groupe chargé ou non chargé, de préférence un groupe -(CH₂)ⱼCOOR₁₃, -(CH₂)ⱼSO₂R₁₃, -(CH₂)ⱼNH₂, -(CH₂)ⱼ-amidino, -(CH₂)ⱼ-hydroxyamidino ou -(CH₂)ⱼ-guanidino avec j = 0, 1 ou 2, R₁₃ représentant un atome d'hydrogène ou un radical alkyle comportant, de préférence, de 1 à 6 atomes de carbone, en particulier un radical éthyle;
et dans laquelle P₁ se présente, dans la formule I, dans une configuration L;
R₄ représente - (CH₂)_{f}OR₁₁ avec f = 1, R₁₁ représentant un atome d'hydrogène et P₂ se présentant, dans la formule I, dans une configuration D;
U est un radical phényle;
Z apparaît en position 3 ou 4 et représente un groupe aminométhyle, guanidino ou amidino 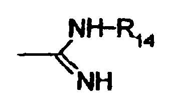 dans lequel
R₁₄ représente un atome d'hydrogène, OH, NH₂, -COR₁₅ ou -COOR₁₅, R₁₅ étant un radical alkyle ramifié ou non ramifié comportant de 1 à 16, de préférence de 1 à 8, en particulier de 1 à 4, en particulier de 1 à 2 atomes de carbone ou bien un radical aryle ou hétéroaryle, aralkyle ou hétéroaralkyle substitué ou non substitué, le radical alkyle possédant de préférence de 1 à 16, en particulier de 1 à 8, en particulier de 1 à 4 et de manière particulièrement préférée de 1 à 2 atomes de carbone et le radical aryle ou hétéroaryle de préférence de 4 à 14, en particulier de 6 à 10, en particulier 6 atomes de carbone et de préférence de 1 à 3 N en tant qu'hétéroatomes;
**caractérisé en ce que**
un ou plusieurs radicaux chargés, de préférence dérivant de -COOH, -CH(COOH)₂, -SO₂H, NH₂, d'un groupe amidino, hydroamidino, amidrazono ou guanidino sont présents dans les radicaux R₂ ou R₅;
ou bien un composé de formule générale 1 sous forme de promédicament ou de son sel.

2. Composé selon la revendication 1, dans lequel une chaîne oligo ou polyalkylène glycol, fonctionnalisée avec un groupe amino ou carboxyle, en particulier une chaîne poly ou oligoéthylène glycol ou poly ou oligopropylène glycol est couplée en plus directement sur un groupe fonctionnel de R₂ en particulier par l'intermédiaire d'un groupe -NH ou -CO en formant une liaison amide sur R₂, la chaîne oligo ou polyalkylène glycol présentant, du moins sur les deux extrémités, un groupe fonctionnel, en particulier un groupe amino et/ou carboxyle substitué ou non substitué ou la chaîne oligo ou polyalkylène glycol présentant, du moins sur une extrémité, un groupe fonctionnel, en particulier un groupe amino et/ou carboxyle substitué ou non substitué et se présente sur l'autre extrémité sous forme d'éther alkylique comportant de 1 à 4 atomes de carbone, en particulier sous forme d'éther méthylique,
R₂ représentant, de préférence,
(a) -(CH₂)ₙ-NH₂ avec n = 1 à 5, de préférence 4 ou
(b) -(CH₂)ₙ-COOH avec n = 1 à 5, de préférence 1 à 3.

3. Composé selon la revendication 1 ou la revendication 2, dans lequel P1, après le couplage de l'oligo ou polyalkylène glycol, répond à la formule générale II 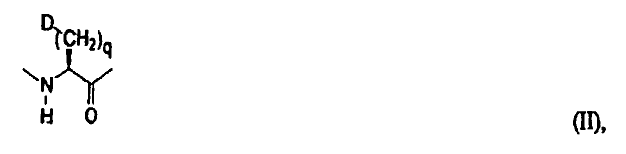 dans laquelle q = 0, 1, 2, 3, 4 ou 5 et D est égal à la formule III
E-F-G- (III),
dans laquelle
-si E est un groupe H₂N, HOOC-(CH₂)ₙ-CO-NH, HOOC ou H₂N-(CH₂)ₙ-NH-CO, F est un oligo- ou polyalkylène glycol de formule générale
-(CH₂)_{d}-[O-CH₂-CH₂]ᵥO-(CH₂)ₘ-(NH-CO-CH₂-O-CH₂)ₖ ou
-(CH₂)_{d}-[O-CH(CH₃)-CH₂]ᵥ-O-(CH₂)ₘ-(NH-CO-CH₂-O-CH₂)k
avec
d = 1, 2, 3 ou 4,
v = un nombre entier de 1 à 1000, de préférence de 1 à 250,
m = 0, 1, 2, ou 4 et
k = 0 ou 1 ou
-si E est un groupe CH₃-O-, F est égal à une chaîne oligo- ou polyalkylène glycol de formule générale
-(CH₂)_{d}-[O-CH₂-CH₂]ᵥO-(CH₂)ₘ-(NH-CO-CH₂-O-CH₂)ₖ ou
-(CH₂)_{d}-[O-CH(CH₃)-CH₂]ᵥ-O-(CH₂)ₘ-(NH-CO-CH₂-O-CH₂)k
avec
d = 1, 2, 3 ou 4,
v = un nombre entier de 1 à 1000, de préférence de 1 à 250,
m = 0, 1, 2, 3 ou 4 et
k = 0 ou 1;
et G est -CO-NH- ou -NH-CO-.

4. Composé selon au moins l'une quelconque des revendications 1 à 3, dans lequel U est substitué sur 1, 2 ou 3 positions, de préférence avec un halogène, en particulier du fluor ou du chlore, ou bien un radical méthyle, éthyle, propyle, méthoxy, éthoxy ou propoxy.

5. Composé selon au l'une quelconque des revendications 1 à 4, dans lequel un groupe carboxyle se présente sous forme d'ester, de préférence d'ester éthylique, protégé.

6. Composé selon au moins l'une quelconque des revendications 1 à 5 sous forme de promédicament, dans lequel R₉ et/ou R₁₁ représente, dans ce cas, un radical alkylcarbonyle, aralkylcarbonyle, alkyloxycarbonyle ou aralkyloxycarbonyle, le radical alkyle possédant de préférence de 1 à 6, en particulier de 1 à 4 atomes de carbone et le radical aryle possédant de préférence de 5 à 8, en particulier 6 atomes de carbone.

7. Composé selon au moins l'une quelconque des revendications 1 à 6, **caractérisé en ce que**
- dans le radical amidinobenzylamide le groupe amidino est en position 4 et P₂ provient de l'acide aminé D-Ser et P₁ de la glycine, de l'alanine, de la sérine, de l'acide asparagique ou de l'acide glutaminique et
- R₅ est un radical aryle ou aralkylsulfonyle non substitué ou pourvu d'un groupe carboxyle ou carboxyalkyle, comportant de 1 à 16, de préférence de 1 à 8, en particulier de 1 à 4, en particulier de 1 à 2 atomes de carbone dans le radical alkyle et de 6 à 14, de préférence de 6 à 10, en particulier 6 atomes de carbone dans le radical aryle.

8. Composé selon au moins l'une quelconque des revendications 1 à 6, **caractérisé en ce que** dans le radical amidinobenzylamide, le groupe amidino est en position 4 et P₂ représente l'acide aminé D-Ser et P₁ un acide aminé basique naturel ou synthétique, non substitué ou substitué dans la configuration L, par exemple Lys, homoLys, Arg, norArg, homoArg, His, Orn, Orn(2-imidazolidinyle), Dab, acide 4-[(2-amino)pyrimidinyl)butyrique, Dap, Ala[3-(2-pyrrolidinyle)], Ala[3-pyrrolidinyle-(2(N-amidino)], Ala[3-(N-pipérazine-4-N-amidino], Ala-4-pip), Ala[4-pip(N-amidino)], homoAla(4-pip), Ala[3-pip(N-amidino)], homoAla(3-pip), homoAla[4-pip-N-amidino)], Ala-(3-guanidino), Phe(3-amidino), Phe(4-amidino), Phe(3-NH₂), Phe(4-NH₂), Phe(3-guanidino), Phe(4-guanidino), Phe[4-(2-imidazolidinyle)], Phe[3-CH2-(guanidino)], Phe[4-CH₂-(guanidino)], homoPhe(3-amidino), homoPhe(4-amidino), hPhe(3-NH₂), hPhe(4-NH₂), hPhe(3-guanidino), hPhe(4-guanidino), cis-Cha(4-NH₂), trans-Cha(4-NH₂), cis-homoCha(4-NH₂), trans-homoCha(4-NH₂), trans-Cha(4-CH₂NH₂), trans-homoCha(4-CH₂NH₂)
et **caractérisé en ce que** R₅ est un radical aryle ou aralkylsulfonyle, pourvu d'un groupe sulfonyle, comportant de 1 à 16, de préférence de 1 à 8, en particulier de 1 à 4, en particulier de 1 à 2 atomes de carbone dans le radical alkyle et de 6 à 14, de préférence de 6 à 10, en particulier 6 atomes de carbone dans le radical aryle lié sur le groupe amino de D-Ser.

9. Composé selon la revendication 8, **caractérisé en ce que** P₁ représente l'acide aminé Lys ou Arg.

10. Composé selon au moins l'une quelconque des revendications 1 à 9, **caractérisé en ce que** le substituant sur le radical aryle, hétéroaryle, aralkyle ou hétéroaralkyle substitué est un halogène, de préférence du fluor, du chlore ou du brome, en particulier du fluor ou du chlore.

11. Composé selon au moins l'une quelconque des revendications 1 à 10, **caractérisé en ce qu'**un composé de formule générale 1 a la structure suivante: 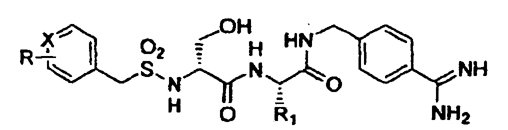 avec
R = COOH, HOOC-(CH₂)ₚ-, R₁₈OOC-(CH₂)ₚ- avec p = 1 et 2 et R₁₈ = un radical méthyle ou éthyle ou un groupe COOMe en position ortho, méta ou para, ou H et X = CH et R₁ = H;
ou
R = 4-COOH ou 3-COOH avec X = CH et R₁ = H, CH₃ ou CH₂-OH; ou
R = 4-COOH ou 3-COOH avec X = CH et R₁ = H, CH₃ ou CH₂-OH; ou
R = 4-CN avec X = CH et R₁ = CH₃; ou
R = 4-(NH₂-CH₂) avec X = CH et R₁ = H; ou
R = 4-COOMe avec X = CH et R₁ = CH₂-OH.

12. Composé selon au moins l'une quelconque des revendications 1 à 10,
**caractérisé en ce qu'**un composé de formule générale I a l'une des structures suivantes: 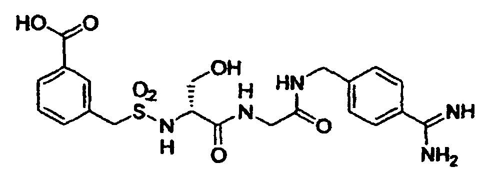 ou 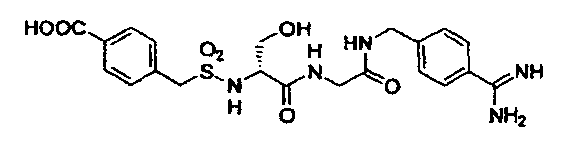 ou

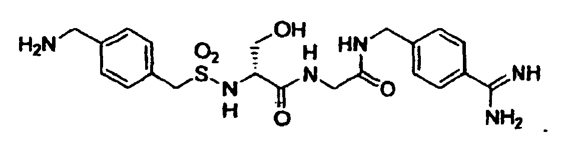

13. Composé selon au moins l'une quelconque des revendications 1 à 10, **caractérisé en ce qu'**un composé de formule générale I a l'une des structures suivantes: 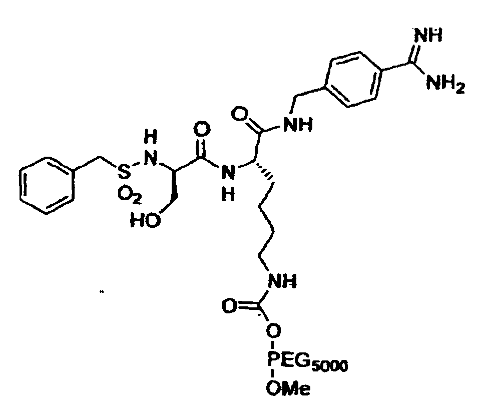 ou 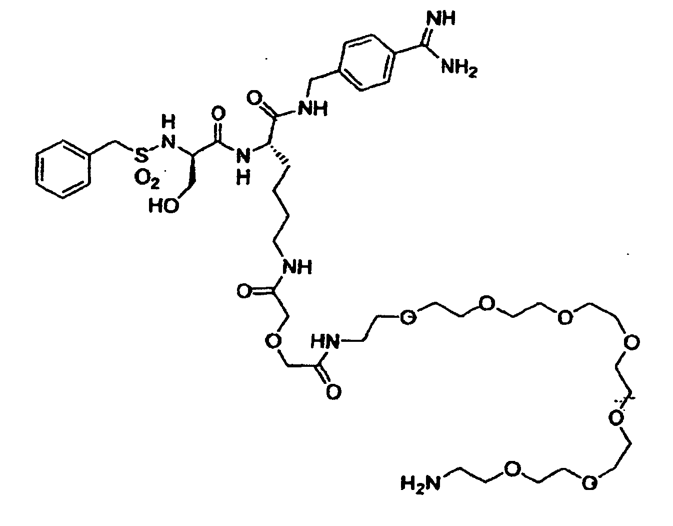 ou 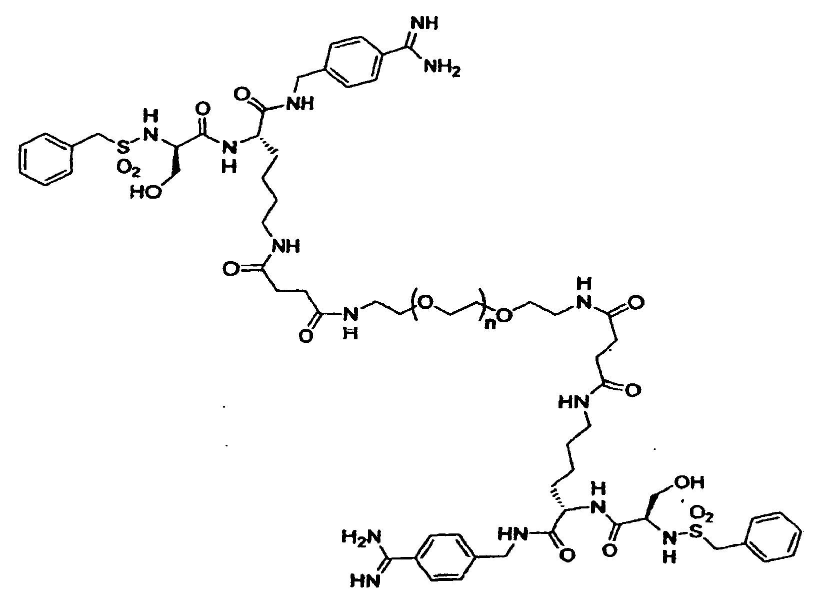 avec n = 2 à 250.

14. Composé selon au moins l'une quelconque des revendications 1 à 13, **caractérisé en ce que** les composés se présentent, de préférence, sous forme de sels, de préférence avec des acides minéraux, de préférence sous forme d'chlorhydrates ou, de préférence, de sels avec des acides organiques appropriés.

15. Composé selon la revendication 14, **caractérisé en ce que** des sels d'acides minéraux préférés sont aussi des sulfates et des acides organiques appropriés par exemple l'acide acétique, l'acide formique, l'acide méthylsulfonique, l'acide succinique, l'acide malique ou l'acide trifluoroacétique, les sels d'acides organiques préférés étant des acétates.

16. Procédé de préparation d'un composé selon au moins l'une quelconque des revendications 1 à 15, **caractérisé en ce que** séquentiellement, les acides aminés correspondants sont couplés sur une 4-acétyloxamidinobenzylamine, l'acide aminé N-terminal portant déjà le radical R5 ou étant ensuite lié à celui-ci.

17. Médicament contenant un composé selon au moins l'une quelconque des revendications 1 à 15, ainsi qu'excipients et/ou additifs adaptés au plan pharmaceutique.

18. Médicament selon la revendication 17, utilisé sous forme de comprimé, de dragée, de capsule, de pellet, de suppositoire, de solution, en particulier de solution pour injection ou transfusion, de gouttes oculaires, nasales ou auriculaires, de sirop, de capsule, d'émulsion ou de suspension, de granules, de stylets, d'aérosol, de poudre, de pâte, de crème ou de pommade.

19. Utilisation d'un composé selon au moins l'une quelconque des revendications 1 à 15 pour fabriquer un médicament pour traiter ou lutter contre une tumeur, en particulier pour réduire la formation de métastases tumorales, de préférence sous forme orale, sous-cutanée, intraveineuse ou transdermique.

20. Utilisation d'un composé selon au moins l'une quelconque des revendications 1 à 15 pour fabriquer un produit de diagnostic d'une tumeur.
